# EUROPEAN PATENT APPLICATION

(11) **EP 3 289 912 A1**
(43) Date of publication of application: **07.03.2018**
(21) Application number: 16786379.4
(22) Date of filing: 20.04.2016
(51) Int. Cl.: A45D 20/12

(54) **LIGHT IRRADIATION DEVICE**

(30) Priority: 27.04.2015 JP 2015089953; 24.12.2015 JP 2015252512
(71) Applicant: Metras, Inc., Chiyoda-ku, Tokyo 100-0006 (JP)
(72) Inventor: SHIIBASHI, Tadashi, Tokyo 100-0006 (JP); ITO, Tomoyuki, Tokyo 100-0006 (JP)
(74) Representative: Noble, Nicholas
(86) International application number: PCT/JP2016/062531
(87) International publication number: WO 2016/175102

(57) **Abstract**

A light irradiation device 100 includes a blower unit 40 that provides an air flow 42 flowing in a first direction P, and a light emitting unit 10 that outputs luminous flux 28 to irradiate a scalp or hair as an irradiation target part 70. The light emitting unit 10 is provided on the first direction P side of the blower unit 40. The light emitting unit 10 includes a cover member 18 that transmits the luminous flux 28 and that is replaceably provided.

## Description

### [TECHNICAL FIELD]

The present invention relates to a light irradiation device that emits light to irradiate the scalp or hair of a user.

### [BACKGROUND ART]

There has been conventionally known a hair dryer that irradiates facial skin with light of a predetermined kind for sterilization of the skin surface, for example. Patent Document 1 describes a hair dryer that provides warm air through an air outlet and comprises a light source for emitting blue light in the direction of the airflow, for example. Meanwhile, there has been studied a technique for irradiating the scalp or hair (hereinafter, referred to as scalp or the like) of a human with light of a predetermined kind in order to activate blood flow in subcutaneous tissue of the scalp and stimulate hair growth.

### [PRIOR ART REFERENCE]

### [PATENT DOCUMENT]

[Patent Document 1] Japanese Unexamined Patent Application Publication No. 2014-128524

### [DISCLOSURE OF INVENTION]

### [PROBLEM(S) TO BE SOLVED BY THE INVENTION]

The hair dryer described in Patent Document 1 includes an air inlet and an air outlet, and air drawn in through the air inlet is heated by a heater provided on an airflow passage within the body case and then blows out of the air outlet as warm air. Further, an ion generator is provided so as to provide ion-containing air, and a light source for emitting blue light in the direction of the airflow is also provided. A hair dryer of this kind includes an LED that emits blue light having a wavelength from 465 nm to 470 nm, thereby killing normal inhabitants of skin existing in the hair. The light source is provided so as to emit blue light in the direction of the airflow. The blue light provides a sterilization effect for reducing or slowing the growth of bacteria, thereby inhibiting propagation of normal inhabitants of skin in the hair irradiated with the blue light even when the hair is moisturized by ion-containing air.

Meanwhile, there has been studied a technique of a light irradiation device that irradiates the scalp or hair of a human with light of a predetermined kind in order to activate blood flow in subcutaneous tissue of the scalp and stimulate hair growth. With regard to such a technique, it has been found that using a certain amount of red light, which has a significant effect on subcutaneous tissue, is effective. Namely, in comparison with the light for sterilization of skin surfaces, a greater amount of light needs to be delivered to the scalp or the like in the technique.

In the hair dryer described in Patent Document 1, since the LED light source of blue light is provided at an end of the body case adjacent to the air outlet, the distance between the LED light source and the scalp or the like to be irradiated is longer than the distance between the air outlet and the scalp or the like. When the distance from the LED light source is longer, the amount of light delivered to the scalp or the like is reduced, which may cause a problem of difficulty in obtaining sufficient effect.

Also, when the distance between the LED light source and the scalp or the like is longer, the range of diffusion of the light from the LED light source becomes greater, so that the light is more likely to enter an eye. If the blue light emitted by the LED light source enters an eye, the eye may be subjected to certain stress due to the so-called blue light problem, and it cannot be said that the long-term influence exerted by such stress has been fully examined.

In the hair dryer described in Patent Document 1, if the emission intensity of the LED light source is raised in order to increase the amount of light delivered to the scalp or the like, the amount of light that enters an eye will also be increased. Namely, in a conventional light irradiation device, increasing the amount of light delivered to the scalp or the like is inconsistent with reducing the influence of light that enters an eye.

The present invention has been made in view of such a problem, and a purpose thereof is to provide a technique of a light irradiation device for irradiating the scalp or the like of a user with light, in which the amount of light delivered to the scalp or the like can be increased while the influence of light that enters an eye is reduced.

### [MEANS TO SOLVE THE PROBLEM(S)]

To solve the problem above, a light irradiation device according to one embodiment of the present invention includes a blower unit that provides an air flow flowing in a first direction, and a light emitting unit that outputs luminous flux to irradiate a scalp or hair as an irradiation target part. The light emitting unit is provided on the first direction side of the blower unit.

According to the embodiment, the light emitting unit is provided forward of the blower unit in the light irradiation device for irradiating a scalp or the like, so that the light emitting unit is brought closer to the scalp or the like and an increased amount of light is delivered to the scalp, thereby reducing the relative proportion of the amount of light that enters an eye.

### [ADVANTAGEOUS EFFECTS OF INVENTION]

The present invention provides a technique of a light irradiation device for irradiating the scalp or the like of a user with light, in which the amount of light delivered to the scalp or the like can be increased while the influence of light that enters an eye is reduced.

### [BRIEF DESCRIPTION OF DRAWINGS]

Embodiments will now be described, by way of example only, with reference to the accompanying drawings which are meant to be exemplary, not limiting, and wherein like elements are numbered alike in several Figures, in which:
Fig. 1 is an exploded perspective view of a light irradiation device according to an embodiment;
Fig. 2 is a front view of the light irradiation device according to the embodiment;
Fig. 3 is a sectional side view of the light irradiation device according to the embodiment;
Fig. 4 is a front view of a cover member of the light irradiation device according to the embodiment;
Fig. 5 is a side view of the cover member shown in Fig. 4;
Fig. 6 is a rear view of the cover member shown in Fig. 4;
Fig. 7 is a magnified sectional view of the vicinity of the cover member and light emitting elements;
Fig. 8 is a front view of a hood member of the light irradiation device according to the embodiment;
Fig. 9 is a side view of the hood member shown in Fig. 8;
Fig. 10 is a front view of a plurality of light emitting elements of the light irradiation device according to the embodiment;
Fig. 11 is a side view of the plurality of light emitting elements shown in Fig. 10;
Fig. 12 is a magnified sectional view of a first engagement part and a second engagement part shown in Fig. 3;
Fig. 13 is a block wiring diagram of the light irradiation device according to the embodiment;
Fig. 14 is a perspective view of a light irradiation device according to a second embodiment of the present invention;
Fig. 15 is a front view of the light irradiation device according to the second embodiment;
Fig. 16 is a sectional view taken along line A-A in Fig. 2;
Fig. 17 is a side view that shows a state where the light irradiation device of the second embodiment is attached to a hair dryer;
Figs. 18A and 18B are sectional views that each show attachment parts shown in Fig. 17 in detail.;
Figs. 19A and 19B are detailed sectional views that show a first modification of the attachment parts shown in Fig. 17;
Figs. 20A and 20B are detailed sectional views that show a second modification of the attachment parts shown in Fig. 17;
Fig. 21 is an illustrative diagram that shows the direction of light emitted by each LED of the light irradiation device according to the second embodiment;
Fig. 22 is an illustrative side view that shows the state shown in Fig. 21 viewed from a side;
Fig. 23 is a sectional side view that shows an example of a mounting substrate according to the second embodiment;
Fig. 24 is a partial sectional side view that shows a third embodiment of the present invention;
Fig. 25 is an illustrative diagram that shows a fourth embodiment of the present invention and shows a state before a light irradiation device is attached to a facial roller; and
Fig. 26 is a side view that shows a state where the light irradiation device of the fourth embodiment is attached to a facial roller.

### [MODE FOR CARRYING OUT THE INVENTION]

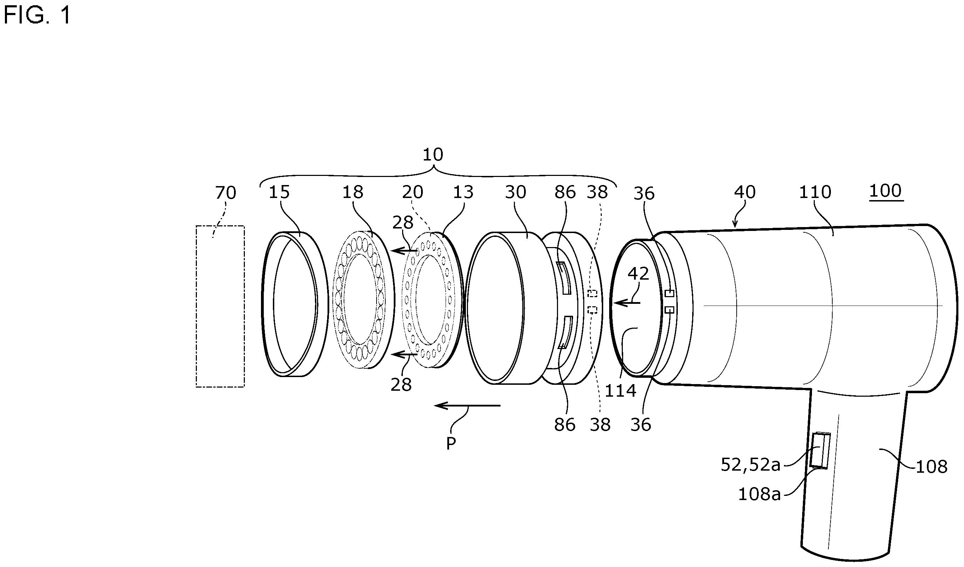

In the following, the present invention will be described based on a preferred embodiment with reference to Figs. 1 through 13. Like reference characters designate like or corresponding constituting elements and members in each drawing, and the same description therefor will be appropriately omitted. Also, the size of a member in each drawing may be appropriately enlarged or reduced in order to facilitate understanding. Further, in each drawing, part of a member less important in describing embodiments may be omitted.

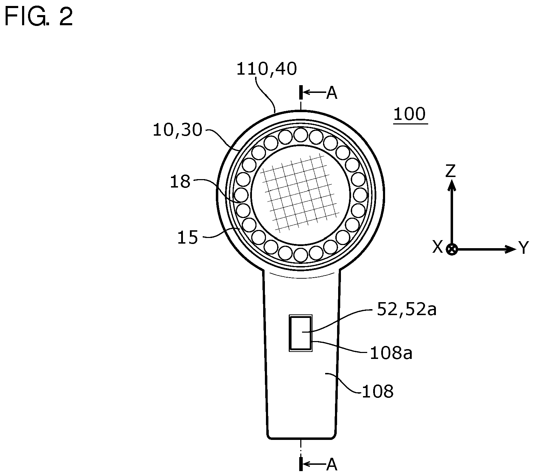

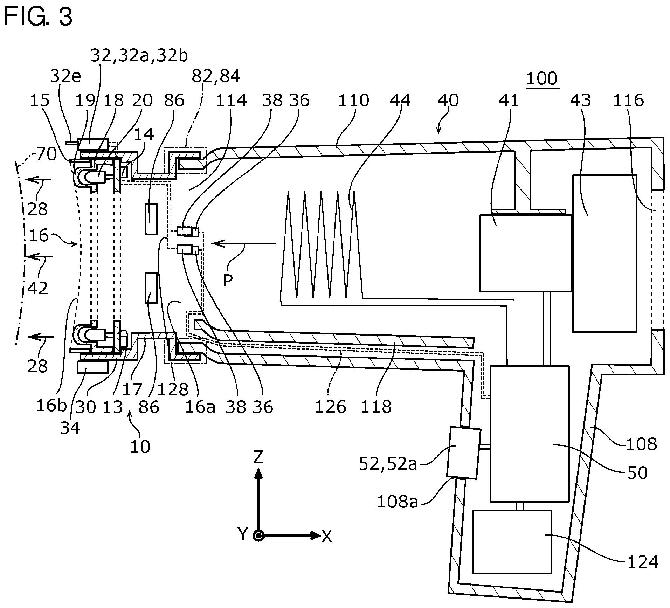

Fig. 1 is an exploded perspective view of a light irradiation device 100 according to an embodiment, Fig. 2 is a front view of the light irradiation device 100, and Fig. 3 schematically shows a side cross section of the light irradiation device 100. In the following, description will be given based on an XYZ Cartesian coordinate system. The direction Y corresponds to a horizontal lateral direction, the direction X corresponds to a horizontal longitudinal direction, and the direction Z corresponds to a vertical direction. Each of the direction Y and the direction Z is perpendicular to the direction X. The specification may also describe the direction Y as the left direction or the right direction, the direction X as the front direction or the back direction, and the direction Z as the upward direction or the downward direction.

As shown in Fig. 3, the light irradiation device 100 according to the embodiment comprises a blower unit 40 that provides an air flow 42 flowing in a first direction P, and a light emitting unit 10 that outputs luminous flux 28 to irradiate a scalp or hair as an irradiation target part 70, and the light emitting unit 10 is provided on the first direction P side of the blower unit 40.

If the irradiation target part 70 is irradiated with the luminous flux 28 while the light irradiation device 100 is placed on the head, the temperature of the irradiation target part 70 may rise. It can be also considered that the light irradiation device 100 itself generates heat during its use and raises the temperature of the irradiation target part 70 accordingly. Therefore, the blower unit 40 in the light irradiation device 100 is configured to provide the air flow 42 toward the irradiation target part 70. By providing the irradiation target part 70 with the air flow 42, temperature rise can be moderated. Further, providing the irradiation target part 70 with non-heated cool air may relax the user and improve blood flow, so that synergistic effect with the luminous flux 28 provided to the scalp can also be expected.

### (Cover Member)

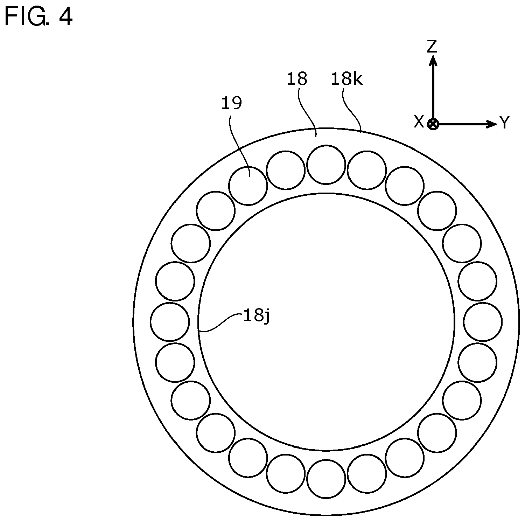

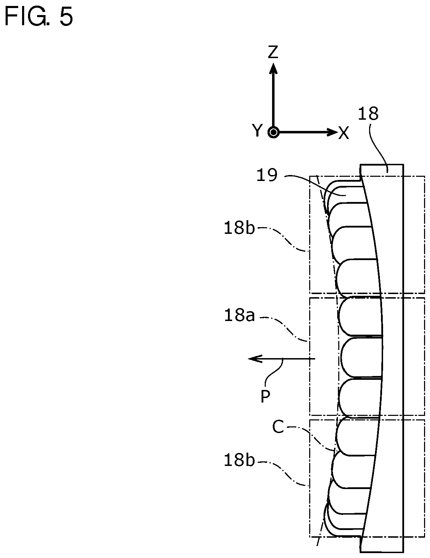

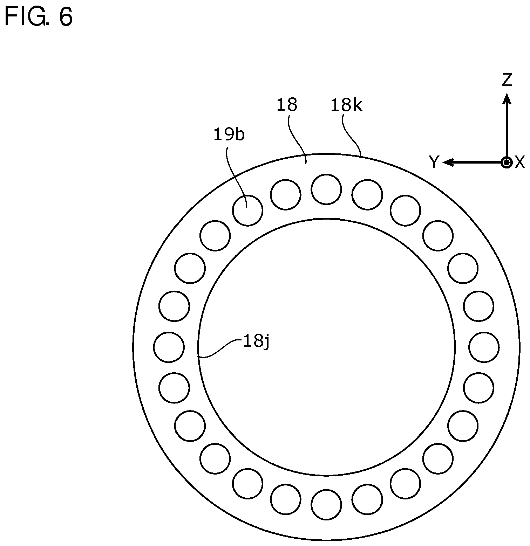

There will now be descried a cover member 18 with reference to Figs. 4 through 6. Fig. 4 is a front view of the cover member 18, Fig. 5 is a side view thereof, and Fig. 6 is a rear view thereof.

If multiple light emitting elements 20 are brought into direct contact with the scalp or hair as the irradiation target part 70, foreign matter, such as sebum, may adhere to the multiple light emitting elements 20, of which the surfaces may be blurred accordingly. If the surfaces of the multiple light emitting elements 20 are blurred, the amount of light to be output could be reduced. Accordingly, it is desirable that the multiple light emitting elements 20 are not brought into direct contact with the irradiation target part 70. Therefore, in the light emitting unit 10 of the light irradiation device 100, the cover member 18, which transmits the luminous flux 28, is replaceably provided. If foreign matter adheres to the cover member 18, the cover member 18 can be detached to be washed. The cover member 18 may be provided with an engagement part, so as to be easily attached and detached.

### (Projections)

If the front surface of the cover member is flat, hair pressed by the cover member will be dense, so that the proportion of luminous flux striking the hair would be increased, whereas the proportion thereof delivered to the scalp would be reduced. Therefore, the cover member 18 of the light irradiation device 100 includes multiple projections 19 that project in the first direction P. Accordingly, the projections 19 thrust through the hair and the tips of the projections 19 come close to the scalp, so that the luminous flux 28 can be efficiently delivered to the scalp. Also, the tips of the projections 19 touching the scalp would provide moderate stimulation to the scalp, thereby stimulating blood flow.

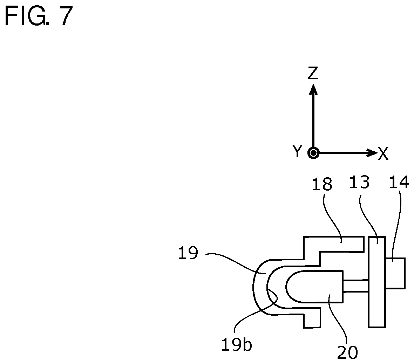

In order to prevent increase in size, the dimension in a longitudinal direction of the light emitting unit may desirably be small. Accordingly, in the light irradiation device 100, the cover member 18 is provided with recesses 19b that respectively house at least part of the multiple light emitting elements 20, as shown in Fig. 7. For example, by allowing the recesses 19b to house the tips of the light emitting elements 20, the dimension in a longitudinal direction of the light emitting unit 10 can be reduced. The recesses 19b may be provided so as to correspond to the projections 19.

It is desirable that the luminous flux 28 contains less ultraviolet light, which is harmful. Therefore, the cover member 18 of the light irradiation device 100 is formed of a material that attenuates ultraviolet light. Accordingly, since the luminous flux 28 that has penetrated the cover member 18 contains less ultraviolet light, the user can safely use the light irradiation device 100.

Since a human head has a substantially spherical shape, the irradiation target part may be often curved. Accordingly, the shape of the cover member may desirably be set to follow the curved shape of the irradiation target part. Therefore, the cover member 18 of the light irradiation device 100 includes a receding region 18a that is receding toward the blower unit 40 along the longitudinal direction of the first direction P, and a projecting region 18b that is projecting forward of the receding region 18a in the first direction P, as shown in Fig. 5. Accordingly, the receding region 18a and the projecting region 18b of the cover member 18 can be arranged so as to follow a curved shape C of an irradiation target part 70.

### (Hood Member)

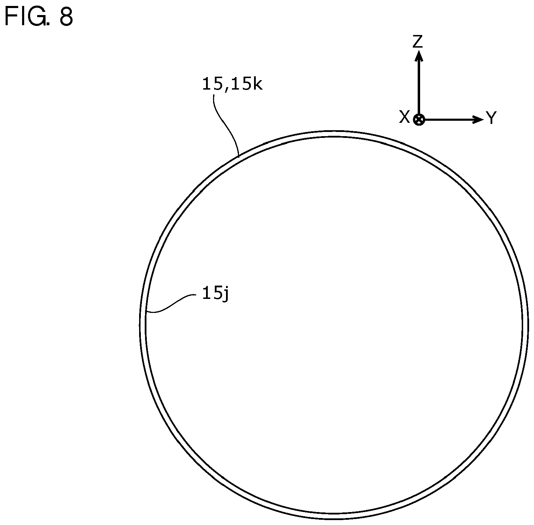

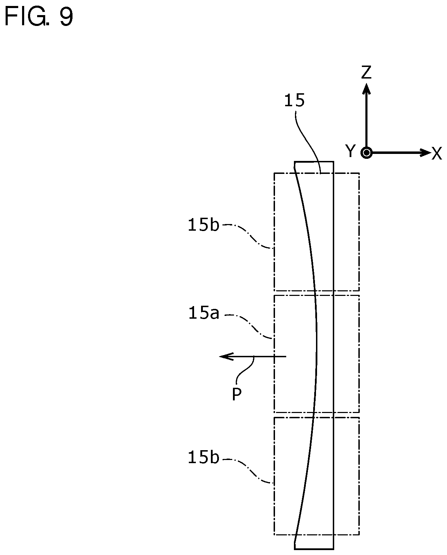

Next, a hood member 15 will be described with reference to Figs. 8 and 9. Fig. 8 is a front view of the hood member 15, and Fig. 9 is a side view thereof.

If part of the luminous flux 28 leaks outside, the leaked light may enter an eye. Therefore, the light emitting unit 10 of the light irradiation device 100 comprises the hood member 15 that surrounds the luminous flux 28 on the first direction P side. With the hood member 15, the amount of light that leaks outside can be reduced. By setting the hood member 15 to be contactable with the irradiation target part 70, the amount of light that leaks outside can be further reduced. The hood member 15 may be provided so as to extend forward of the projections 19 of the cover member 18. The hood member 15 includes a receding region 15a corresponding to the receding region 18a of the cover member 18, and a projecting region 15b corresponding to the projecting region 18b of the cover member 18. The projecting region 15b is positioned forward of the receding region 15a in the first direction P.

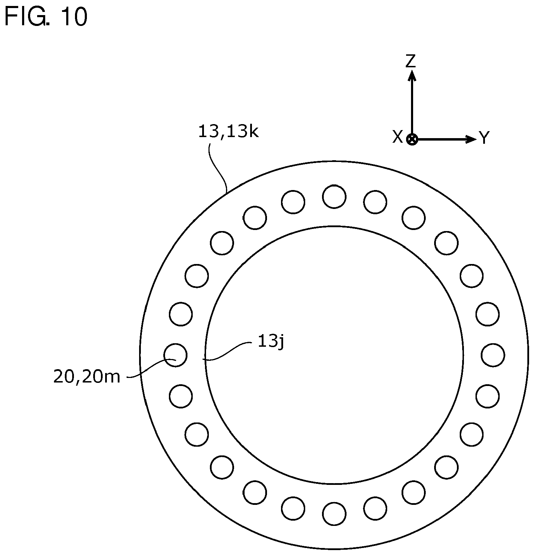

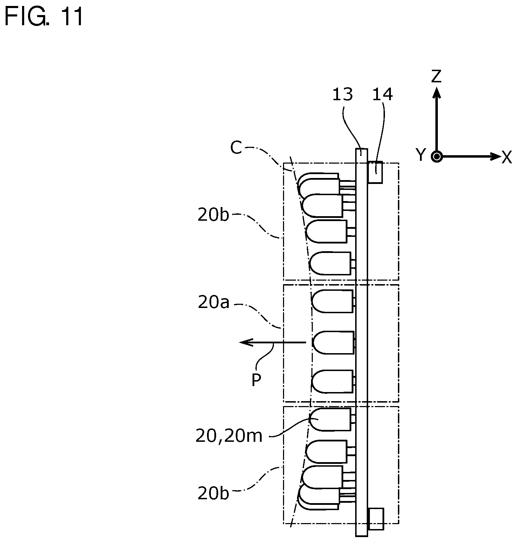

There will now be described the multiple light emitting elements 20 with reference to Figs. 10 and 11. Fig. 10 is a front view of the multiple light emitting elements 20 attached to a substrate 13, and Fig. 11 is a side view thereof.

In terms of users' convenience, the light irradiation device may desirably be capable of emitting luminous flux in a wider range. Accordingly, the light emitting unit 10 of the light irradiation device 100 includes the multiple light emitting elements 20, as shown in Fig. 10. As the multiple light emitting elements 20, light emitting diodes (LEDs) or laser diodes (LDs) may be used, for example. In the light irradiation device 100, the multiple light emitting elements 20 are arranged so as to surround the air flow 42. The multiple light emitting elements 20 may be arranged in a circular, an oval, or a polygonal shape to surround the air flow 42.

As stated previously, since a human head has a substantially spherical shape, the irradiation target part may be often curved. Accordingly, the position of each of the light emitting elements may desirably be set to follow the curved shape of the irradiation target part. Therefore, the multiple light emitting elements 20 of the light irradiation device 100 include light emitting elements 20a that are receding toward the blower unit along the longitudinal direction of the first direction P, and light emitting elements 20b that are projecting forward of the receding light emitting elements 20a in the first direction, as shown in Fig. 11. Namely, in the light irradiation device 100, the position in the longitudinal direction of each of the multiple light emitting elements 20 is adjusted so as to follow a curved shape C of a head. In the light irradiation device 100, as an example, along the direction Z (vertical direction), light emitting elements 20a near the center are arranged to recede along the longitudinal direction of the first direction P backward of light emitting elements 20b on the both sides (upper side and lower side). Accordingly, differences in distance from the surface of a head among the multiple light emitting elements 20 become smaller, so that the luminous flux 28 can be effectively provided.

The amount of light received at each region of the irradiation target part may desirably be less different from each other. Accordingly, in the light irradiation device 100, at least part of the multiple light emitting elements 20 are provided to be inclined with respect to the longitudinal direction of the first direction P. Namely, at least part of the multiple light emitting elements 20 of the light irradiation device 100 are provided so that the optical axes thereof are inclined with respect to the longitudinal direction. In the light irradiation device 100, as an example, the multiple light emitting elements 20 are inclined inward so that the optical axes thereof intersect in front. Alternatively, the multiple light emitting elements 20 may be inclined outward so that the optical axes thereof are directed away from each other in front. The direction of the inclination may be set according to the directivity of the light emitting element. The inclination may be set within the range of 3 to 30 degrees, for example. In the light irradiation device 100, the multiple light emitting elements 20 are provided so that the optical axes thereof are inclined at 3 degrees inward with respect to the longitudinal direction.

When luminous flux with a greater amount of light is output, temperature rise may occur in the light emitting elements of the light emitting unit or electronic components therearound. If the operating temperature is higher, the life of the light emitting elements and electronic components will be shortened, so that it is desirable to use the light emitting elements and the electronic components therearound while cooling them. Accordingly, in the light irradiation device 100, at least part of the multiple light emitting elements 20 are arranged in a region where the air flow 42 passes through, as shown in Fig. 3. Namely, the multiple light emitting elements 20 are arranged in a range extending forward from an air outlet 114. In this case, the multiple light emitting elements 20 are cooled by the air flow 42 provided through the air outlet 114, so that temperature rise can be prevented.

Also, in the light emitting unit 10 of the light irradiation device 100, at least part of electronic components 14, each electrically connected to one of the multiple light emitting elements 20, are arranged in a range where the air flow 42 passes through, as shown in Fig. 3. Namely, at least part of the electronic components 14 are arranged so that heat dissipation is promoted by the air flow 42. Accordingly, the electronic components 14 are exposed to the air flow 42 and cooled thereby, so that temperature rise can be prevented.

It is desirable to reduce the possibility that the luminous flux 28 output by the light emitting unit 10 will enter an eye. Accordingly, as shown in Fig. 3, the light irradiation device 100 comprises a current limiting unit 32 configured to shut off or reduce the current supplied to the light emitting unit 10 when the light emitting unit 10 is apart from the irradiation target part 70 by a predetermined distance or more. Namely, the light irradiation device 100 is configured so that, when the light emitting unit 10 is close to the irradiation target part 70, the light emitting unit 10 is energized and outputs the luminous flux 28, and, when the light emitting unit 10 is distanced from the irradiation target part 70, the energization is substantially stopped. For example, the current limiting unit 32 may be configured to detect the distance from the light emitting unit 10 and control energization of the light emitting unit 10 according to the detected distance. Also, the current limiting unit 32 may be configured to energize the light emitting unit 10 when the light emitting unit 10 is brought into contact with the irradiation target part 70 or the vicinity thereof. Accordingly, when the light emitting unit 10 is distanced from the head, the output of the luminous flux 28 is automatically decreased, thereby reducing the possibility that the luminous flux 28 enters an eye. In order to facilitate understanding, illustration of the current limiting unit 32 is omitted in Figs. 1 and 2.

### (Sign Output Unit)

For example, a head may be separated into multiple irradiation target parts, and the light irradiation device may be used for each of the irradiation target parts for a predetermined period of time. In terms of usability, the operating time for each of the irradiation target parts may be suitably set within the range of 3 to 30 seconds, and more suitably set within the range of 5 to 20 seconds. The operating time for each of the irradiation target parts will be shorter when the luminous flux from the light irradiation device is larger, and will be longer when the luminous flux is smaller. It will be convenient for a user if a sign for the operating time is output. Accordingly, as shown in Fig. 3, the light irradiation device 100 comprises a sign output unit 34 configured to output a sign that can be perceived by a user at predetermined timing. As an example, the sign output unit may be configured to output a beep 34e as a sign every preset time (10 seconds, for example). Accordingly, the user can change the irradiation position at the timing of the sign during use. The sign is not particularly limited, as long as it can be perceived by users. In order to facilitate understanding, illustration of the sign output unit 34 is omitted in Figs. 1 and 2.

The operating time for each of the irradiation target parts may desirably be constant. Accordingly, in the light irradiation device 100, the sign output unit 34 is configured to output a sign using at least one of sound, vibration, and light, at timing when a predetermined period of time elapses after the supply of a current to the light emitting unit 10 is started. As an example, the sign output unit 34 may be configured to output the beep 34e as a sign, at timing when a preset time (5 seconds, for example) elapses after the supply of a current to the light emitting unit 10 is started. Accordingly, the user can change the irradiation position at the timing of the beep 34e during use. In this case, the operating time for each of the irradiation target parts is likely to be constant.

If the light irradiation device can be decomposed into blocks, it can be compactly stored and will be convenient for carrying. Meanwhile, it is desirable that, only by supplying power to one of the light emitting unit and the blower unit, the other can also be supplied with power in the light irradiation device. Accordingly, in the light irradiation device 100, the light emitting unit 10 is provided to be attachable to and detachable from the blower unit 40, the blower unit 40 includes a first contact part 36 used to supply a current to the light emitting unit 10, and the light emitting unit 10 includes a second contact part 38 that is electrically connected to the first contact part 36 when the light emitting unit 10 is attached to the blower unit 40. With such a configuration, the light irradiation device 100 can be decomposed into the light emitting unit 10 and the blower unit 40 to be stored. Also, by attaching the light emitting unit 10 to the blower unit 40 and supplying power only to the blower unit 40 in the light irradiation device 100, the light emitting unit 10 can also be supplied with power via the first contact part 36 and the second contact part 38.

There will now be described the specific structure of the light irradiation device 100.

### (Blower Unit)

As shown in Fig. 3, the blower unit 40 comprises a casing 110, a grip portion 108, an air inlet 116, the air outlet 114, an impeller 43, a motor 41, a heater 44, a control unit 50, a switch unit 52, a wiring passage unit 118, the first contact part 36, a power supply unit 124, and a first engagement part 82.

The casing 110 is a housing that houses and retains therein the main constituting elements of the blower unit 40. The casing 110 is a hollow and substantially-cylindrical member, which can be formed of a resin material through a molding process. The grip portion 108 is a part held by a user with a hand and attached so as to protrude downward from the lower surface of the outer periphery of the casing 110 near the rear side. The grip portion 108 may be connected to the casing 110 via a hinge means for enabling folding.

The air inlet 116 is an opening through which air to be provided is introduced from the surrounding space into the casing 110, and may be provided on the rear side of the casing 110, for example. The air inlet 116 may be provided with a mesh member (not illustrated) to inhibit entry of foreign matter. The air outlet 114 is an opening through which the air flow 42 comes out and provided on the front side of the casing 110. The air outlet 114 may also be provided with a mesh member (not illustrated) to inhibit entry of foreign matter.

The impeller 43 is a blade member that rotates to push air at the back toward the front, and can be formed of a resin material through a molding process, for example. The impeller 43 is disposed in front of the air inlet 116 within the casing 110. The motor 41 is an electric motor for rotating the impeller 43 and rotates with a current supplied from the control unit 50. The motor 41 is disposed in front of the impeller 43 within the casing 110, and the rotating shaft (not illustrated) of the motor 41 is fixed to the center of the impeller 43.

The heater 44 is an electric heater for heating the air flow pushed out by the impeller 43, and generates heat with a current supplied from the control unit 50. The heater 44 is disposed in front of the motor 41 within the casing 110. The control unit 50 is configured to control electric power supplied from the power supply unit 124 and supply a current to each of the motor 41, heater 44, and light emitting unit 10 according to the state of the switch unit 52. The control unit 50 may be housed and fixed within the grip portion 108, for example. Also, the control unit 50 may be housed within the casing 110. In order to restrict excessive irradiation for the irradiation target part 70, there may be provided a means for limiting the amount of light emitted by the light emitting unit 10. As an example, the control unit 50 may be configured to limit power supply to the light emitting unit 10, based on a value obtained through a predetermined calculation using, as parameters, a current supplied to the light emitting unit 10 and the time for which the current is supplied.

The switch unit 52 is a member for switching the operation mode of the light irradiation device 100. The switch unit 52 may include a mechanical switch having a mechanical contact, and an electronic switch that detects a finger touch and electronically switches the operation mode. The switch unit 52 is attached to the grip portion 108 so that a protruding part 52a protrudes from an opening 108a provided on the front side of the grip portion 108. The switch unit 52 is configured to switch the operation mode among multiple modes when the protruding part 52a is pressed backward. For example, starting from the all-off state, the first press of the protruding part 52a may supply a current to the motor 41 so as to start to provide air, the second press may supply a current to the light emitting unit 10 so as to start light emission, the third press may stop the current supply to the light emitting unit 10 and supply a current to the heater 44 so as to start heating, and the fourth press may place the light irradiation device 100 in the all-off state again. The switch unit 52 may include multiple electrical switches. Also, the switch unit 52 may include a light emission control switch used to start or stop power supply to the light emitting unit 10, and the light emission control switch may be configured to start or stop the power supply according to the orientation of the light irradiation device 100. As an example, the light emission control switch may be configured to start power supply to the light emitting unit 10 when the orientation of the light irradiation device 100 is set to a predetermined orientation (such as an orientation in which the light emitting unit 10 faces a head).

The first contact part 36 has a contact to be electrically connected to the second contact part 38 and is electrically connected to an output part of the control unit 50 via an electric cable 126. The first contact part 36 may be provided on the outer periphery of the first engagement part 82, for example. The wiring passage unit 118 is a passage for protecting the electric cable 126, which connects the control unit 50 and the light emitting unit 10, from the heat generated by the heater 44. Particularly, in order to house the electric cable 126 connecting the control unit 50 and the first contact part 36, the wiring passage unit 118 is provided on the lower part of the casing 110.

The power supply unit 124 is an electrical circuit for providing electric power supplied through a power supply cable (not illustrate) to the control unit 50, and is housed and fixed within the grip portion 108. The power supply unit 124 may include a rectifier circuit and a smoothing circuit.

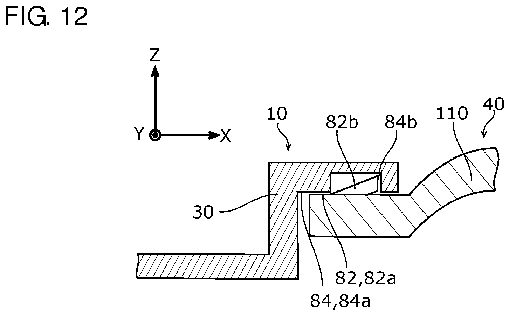

Fig. 12 is a magnified sectional view of the first engagement part 82 and a second engagement part 84, which will be described later. The first engagement part 82 is an attaching/detaching mechanism with which the light emitting unit 10 is detachably mounted to the blower unit 40. The first engagement part 82 includes a fitting outer surface 82a provided on the front part of the casing 110, and a click part 82b provided on the fitting outer surface 82a. The fitting outer surface 82a may be formed to have a diameter smaller than that of a region in the rear of the fitting outer surface 82a, for example. On the fitting outer surface 82a, one or multiple (two, for example) click parts 82b may be provided. The fitting outer surface 82a is configured to fit to a fitting inner surface 84a of the second engagement part 84. The click part 82b is configured to engage with a step part 84b of the second engagement part 84.

### (Light Emitting Unit)

As shown in Fig. 3, the light emitting unit 10 comprises a housing 30, a vent hole 86, the cover member 18, hood member 15, substrate 13, electronic components 14, multiple light emitting elements 20, second contact part 38, and second engagement part 84.

The housing 30 is a hollow and substantially-cylindrical member that houses and retains therein the main constituting elements of the light emitting unit 10. The housing 30 may be formed of a resin material through a molding process, for example. The vent hole 86 is an opening that communicates the inside and the outside of the housing 30, and one or multiple (four, for example) vent holes 86 are formed on the outer periphery of the housing 30. The vent hole 86 can promote air ventilation within the housing 30, thereby preventing temperature rise in the irradiation target part 70.

The cover member 18 is a substantially-discoid member provided perpendicularly to a longitudinal direction (direction X) and includes an inner periphery 18j and an outer periphery 18k, as shown in Figs. 4-7. The cover member 18 may be formed of a resin material having favorable light transmission properties through a molding process, for example. On the front side of the cover member 18, multiple (24, for example) projections 19, which project forward, are provided. Each of the projections 19 has a shell-like shape, such as a cylinder with a hemisphere connected to an end thereof. As shown in Fig. 6, on the back side of the cover member 18, multiple (24, for example) recesses 19b, which are recessed forward, are provided. The recesses 19b house the tips of the multiple light emitting elements 20. The recesses 19b are provided so as to correspond to the projections 19.

The hood member 15 is a hollow cylindrical member extending in a longitudinal direction (direction X) and includes an inner periphery 15j and an outer periphery 15k, as shown in Figs. 8 and 9. The hood member 15 may be formed of a resin material through a molding process, for example. The hood member 15 may be formed of a material having low light transmission properties. On the inner surface of the hood member 15, a reflecting surface may be formed. Further, the hood member 15 may be formed integrally with the cover member 18 so as to be attachable to and detachable from the light emitting unit 10.

The substrate 13 is a printed wiring board of a substantially-discoid shape provided perpendicularly to a longitudinal direction and includes an inner periphery 13j and an outer periphery 13k, as shown in Fig. 10 and 11. The outer periphery 13k of the substrate 13 is fixed to a step part of the housing 30 by screwing or bonding. The electronic components 14 may include a resistor for limiting a current flowing through the multiple light emitting elements 20, or a capacitor for smoothing a voltage. The electronic components 14 are fixed to the front side or the back side of the substrate 13 by soldering, for example.

As shown in Figs. 10 and 11, the multiple light emitting elements 20 include multiple (24, for example) LEDs 20m that are provided on the front side of the substrate 13 to be substantially evenly spaced in a circumferential direction. Although the LEDs 20m are not particularly limited, in the light irradiation device 100 of the embodiment, LEDs that emit light with a wavelength from 620 nm to 670 nm (red) are employed. For example, it has been found that using red light of 638 nm with an amount of 1-1.5 J/cm² is effective for activation of subcutaneous tissue.

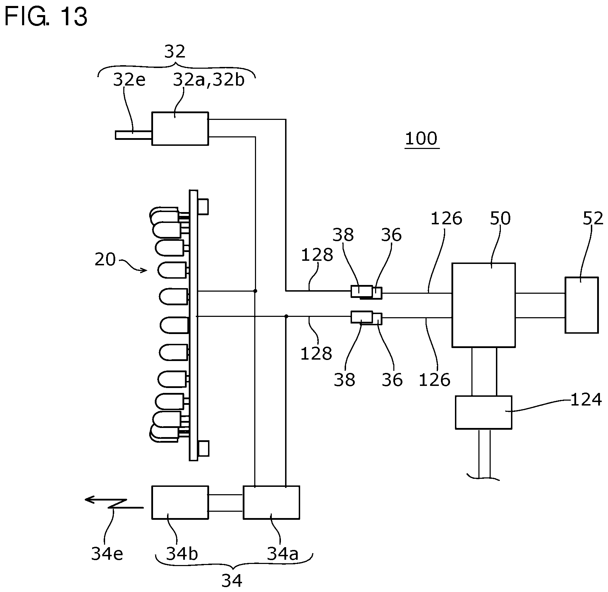

The second contact part 38 has a contact to be electrically connected to the first contact part 36 and is electrically connected to the multiple light emitting elements 20 via an electric cable 128, as shown in Fig. 13. The second contact part 38 may be provided on the inner periphery of the second engagement part 84, for example. When the light emitting unit 10 is attached to the blower unit 40, the two contacts of the second contact part 38 are electrically connected to the two contacts of the first contact part 36, respectively.

As shown in Fig. 12, the second engagement part 84 is an attaching/detaching mechanism with which the light emitting unit 10 is detachably mounted to the blower unit 40. The second engagement part 84 includes the fitting inner surface 84a provided on the rear part of the housing 30, and the step part 84b formed on the fitting inner surface 84a. On the fitting inner surface 84a, one or multiple (two, for example) step parts 84b may be provided. The fitting inner surface 84a is configured to fit to the fitting outer surface 82a of the first engagement part 82. The step part 84b is configured to engage with the click part 82b of the first engagement part 82.

### (Current Limiting Unit)

The current limiting unit 32 is configured to shut off or reduce the current supplied to the light emitting unit 10 when the light emitting unit 10 is apart from the irradiation target part 70 by a predetermined distance or more. The current limiting unit 32 includes a sensor unit 32a and is configured to shut off or reduce the current supplied to the light emitting unit 10 according to the state of the sensor unit 32a. For the sensor unit 32a, a limit switch, of which the output state is changed according to mechanical contact, or a distance sensor using light or sound waves may be employed. In the light irradiation device 100 of the embodiment, the sensor unit 32a is attached to the outer periphery of the housing 30, as shown in Fig. 3. The sensor unit 32a includes a tip part 32e protruding forward on the front part and also contains a limit switch 32b that is turned on when the tip part 32e is brought into contact with an irradiation target part 70 and is pressed into the sensor unit 32a. As shown in Fig. 13, the limit switch 32b, connected in series to the light emitting unit 10, is turned off when the light irradiation device 100 is not used, and is turned on when the tip part 32e is brought into contact with an irradiation target part 70, so that the light emitting unit 10 is energized. By adjusting the position of the tip part 32e, the distance for switching energization and non-energization can be set to a desired distance.

### (Sign Output Unit)

The sign output unit 34 is configured to output a sign using at least one of sound, vibration, and light, at predetermined timing. The sign output unit 34 is provided in the light emitting unit 10 or the blower unit 40. In the light irradiation device 100, the sign output unit 34 is provided on the outer periphery of the housing 30, as shown in Fig. 3. In the light irradiation device 100 of the embodiment, the sign output unit 34 is configured to output a sign at timing when a predetermined period of time elapses after the supply of a current to the light emitting unit 10 is started. As shown in the block wiring diagram of Fig. 13, the sign output unit 34 includes a timer means 34a, of which the output state is changed from OFF to ON when a preset period of time elapses after the energization of the light emitting unit 10 is started, and a buzzer means 34b, which generates the beep 34e when the output state of the timer means 34a is ON. When the light emitting unit 10 is energized, the timer means 34a is activated and starts time counting. After the timer means 34a performs time counting for a predetermined period of time, the buzzer means 34b is energized to generate the beep 34e. When the light emitting unit 10 is not energized, the timer means 34a is reset and the buzzer means 34b is stopped. When the light emitting unit 10 is energized again, the timer means 34a is activated, and the sign output unit 34 repeats the operations described above.

There will now be described a method for using the light irradiation device 100 configured as set forth above.

### (1) First use mode

In the first use mode, the blower unit 40 is not operated, and only the light emitting function of the light emitting unit 10 is used. This mode will be referred to as the first mode. First, the switch unit 52 is operated to place the light irradiation device 100 in the first mode. In this state, the current limiting unit 32 is operated, and the light emitting unit 10 does not emit light. When the light emitting unit 10 is brought closer to a head and the tip part 32e comes into contact with an irradiation target part 70, the light emitting unit 10 is energized and starts light emission to irradiate the irradiation target part 70 with the luminous flux 28. Concurrently, the timer means 34a is activated and, when 10 seconds as preset elapses, for example, the buzzer means 34b generates the beep 34e. In response to the beep 34e, the user will once move the light emitting unit 10 away from the head. In this state, the light emission and the beep 34e is stopped. If the user places the light emitting unit 10 close to an irradiation target part 70 at another position of the head, and the tip part 32e comes into contact with the irradiation target part 70, the same operations as stated above will be repeated. With such repetitive operations, the luminous flux 28 can be provided to a desired range of the head.

### (2) Second use mode

In the second use mode, the blower unit 40 is placed in the state of supplying non-heated cool air and used with the light emitting function of the light emitting unit 10. This mode will be referred to as the second mode. First, the switch unit 52 is operated to place the light irradiation device 100 in the second mode. In this state, a non-heated air flow 42 provided by the blower unit 40 passes through a center hole part 16 of the light emitting unit 10 and then blows out of an outlet 16b. When the light emitting unit 10 is brought close to a head, the head receives the air flow 42. Further, when the light emitting unit 10 is brought closer to the head, and the tip part 32e comes into contact with an irradiation target part 70, the light emitting unit 10 is energized and starts light emission to irradiate the irradiation target part 70 with the luminous flux 28. Thus, in this state, the irradiation target part 70 receives the non-heated air flow 42 and the luminous flux 28 at the same time. As with in the first use mode, the sign output unit 34 is operated, and the beep 34e is generated at predetermined timing. In response to the beep 34e, the user will place the light emitting unit 10 on an irradiation target part 70 at another position of the head, and the same operations as stated above will be repeated. With such repetitive operations, the user can receive the non-heated air flow 42 and the luminous flux 28 at the same time on a desired range of the head.

### (3) Third use mode

In the third use mode, the blower unit 40 is placed in the state of supplying heated warm air and used with the light emitting function of the light emitting unit 10. This mode will be referred to as the third mode. First, the switch unit 52 is operated to place the light irradiation device 100 in the third mode. In this state, a heated air flow 42 provided by the blower unit 40 passes through the center hole part 16 of the light emitting unit 10 and then blows out of the outlet 16b. The operations thereafter are the same as those in the second use mode, and, by repeating the operations, the heated air flow 42 and the luminous flux 28 can be provided at the same time to a desired range of the head.

### (4) Fourth use mode

In the fourth use mode, the light emitting unit 10 is not operated, and only the blower function of the blower unit 40 is used. In this case, the light emitting unit 10 may be detached from the blower unit 40, or may be attached to the blower unit 40. In the following, an example of using the light irradiation device 100 with the light emitting unit 10 attached to the blower unit 40 will be described. This mode will be referred to as the fourth mode. First, the switch unit 52 is operated to place the light irradiation device 100 in the fourth mode. In this state, a non-heated or heated air flow 42 provided by the blower unit 40 passes through the center hole part 16 of the light emitting unit 10 and then blows out of the outlet 16b. According to the fourth use mode, the user can receive the non-heated or heated air flow 42 on a desired range of the head. In the fourth use mode, the sign output unit 34 may be used, or may be not used.

The light irradiation device 100 may be used in the state where at least part of the front part, i.e., the first direction P side, of the light emitting unit 10 is in contact with a head. Particularly, in the first through the third use modes above, the light irradiation device 100 may be used while it is partly in contact with a head. By using the light irradiation device 100 in this way, the amount of light that leaks outside can be reduced.

There will now be described the features of the light irradiation device 100 according to the embodiment of the present invention.

The light irradiation device 100 comprises the blower unit 40 that provides an air flow 42 flowing in the first direction P, and the light emitting unit 10 that outputs the luminous flux 28 to irradiate a scalp or hair as an irradiation target part 70, and the light emitting unit 10 is provided on the first direction P side of the blower unit 40. With such a configuration, the irradiation target part 70 can receive the luminous flux 28 from the light irradiation device 100.

The blower unit 40 in the light irradiation device 100 is configured to provide the air flow 42 toward the irradiation target part 70, which can receive the air flow 42 together with the luminous flux 28 accordingly.

In the light emitting unit 10 of the light irradiation device 100, the cover member 18, which transmits the luminous flux 28, is replaceably provided, thereby reducing the possibility that the multiple light emitting elements 20 are brought into direct contact with the irradiation target part 70.

In the light irradiation device 100, the cover member 18 includes the multiple projections 19 that project in the first direction P, and the projections 19 thrust through hair and can moderate density of the hair. Also, in the light irradiation device 100, the cover member 18 is provided with the recesses 19b that respectively house at least part of the multiple light emitting elements 20, so that the tips of the light emitting elements 20 can be housed by the recesses 19b. Also, since the cover member 18 of the light irradiation device 100 is formed of a material that attenuates ultraviolet light, the cover member 18 can attenuate ultraviolet light in the luminous flux 28.

In the light irradiation device 100, since the cover member 18 includes the receding region 18a, which is receding toward the blower unit 40 along the longitudinal direction of the first direction P, and the projecting region 18b, which is projecting forward of the receding region 18a in the first direction P, the cover member 18 can follow a curved shape of an irradiation target part 70.

The light emitting unit 10 of the light irradiation device 100 comprises the hood member 15 that surrounds the luminous flux 28 on the first direction P side, and the hood member 15 can cover the luminous flux 28.

The light emitting unit 10 of the light irradiation device 100 comprises the multiple light emitting elements 20, so that the light emitting unit 10 can emit light with the multiple light emitting elements 20.

In the light irradiation device 100, since the multiple light emitting elements 20 include the light emitting elements 20a, which are receding toward the blower unit along the longitudinal direction of the first direction P, and the light emitting elements 20b, which are projecting forward of the receding light emitting elements 20a in the first direction, the multiple light emitting elements 20 can follow a curved shape of an irradiation target part 70.

In the light irradiation device 100, at least part of the multiple light emitting elements 20 are provided to be inclined with respect to the longitudinal direction of the first direction P, so that the multiple light emitting elements 20 can include light emitting elements inclined from the first direction P.

In the light irradiation device 100, at least part of the multiple light emitting elements 20 are arranged in a region where the air flow 42 passes through, so that the multiple light emitting elements 20 can include light emitting elements arranged in a region where the air flow 42 passes through.

In the light emitting unit 10 of the light irradiation device 100, at least part of the electronic components 14, each electrically connected to one of the multiple light emitting elements 20, are arranged in a range where the air flow 42 passes through, so that the light emitting unit 10 can include electronic components arranged in a region where the air flow 42 passes through.

Since the light irradiation device 100 comprises the current limiting unit 32 configured to shut off or reduce the current supplied to the light emitting unit 10 when the light emitting unit 10 is apart from the irradiation target part 70 by a predetermined distance or more, the current at the light emitting unit 10 can be changed when the light emitting unit 10 is apart from the irradiation target part 70.

Since the light irradiation device 100 comprises the sign output unit 34 configured to output a sign that can be perceived by a user at predetermined timing, the user can perceive the predetermined timing with the sign.

In the light irradiation device 100, since the sign output unit 34 is configured to output a sign using at least one of sound, vibration, and light, at timing when a predetermined period of time elapses after the supply of a current to the light emitting unit 10 is started, the user can perceive the timing when the predetermined period of time elapses, with the at least one of sound, vibration, and light.

In the light irradiation device 100, since the light emitting unit 10 is provided to be attachable to and detachable from the blower unit 40, the blower unit 40 includes the first contact part 36 used to supply a current to the light emitting unit 10, and the light emitting unit 10 includes the second contact part 38 that is electrically connected to the first contact part 36 when the light emitting unit 10 is attached to the blower unit 40, the light emitting unit 10 of the light irradiation device 100 can be detached.

The present invention has been described with reference to the embodiment. The embodiment is intended to be illustrative only, and it will be obvious to those skilled in the art that various modifications and changes could be developed within the scope of claims of the present invention and that such modifications and changes also fall within the scope of claims of the present invention. Therefore, the description in the present specification and the drawings should be regarded as exemplary rather than limitative.

### (First modification)

Although there has been described an example in which LEDs outputting red light are used in the light irradiation device 100, the application is not limited thereto. For example, LEDs outputting green light or blue light may be used, or LEDs outputting light of assorted colors may be used in combination.

### (Second modification)

Although there has been described an example in which LEDs or LDs are used as the light emitting elements in the light irradiation device 100, the application is not limited thereto. For example, organic EL elements or other kinds of light emitting elements may also be used.

### (Third modification)

The light irradiation device of the present invention may be provided with a means for providing air containing negative ions, or a means for providing a mist obtained by atomizing liquid, such as water and beauty essence.

In the following, light irradiation devices 210, 220, and 230 according to the second through fourth embodiments of the present invention will be described. In recent years, it has been found that light emitted by a light emitting diode (an LED) improves cell activity, has no side effects on cell activity, increases fibroblasts, produces a growth factor, produces collagen, and increases vascularization. Further, it has been found that LED irradiation is effective for healing of skin tissue or hair.

On the basis of such findings, the light irradiation devices 210, 220, and 230 according to the second through fourth embodiments of the present invention are proposed. Each of the light irradiation devices 210, 220, and 230 comprises multiple light emitting diodes (1A: hereinafter, referred to as "LEDs" in this specification) and can be attached to and detached from cosmetic appliances 180 and 280.

The LEDs may suitably emit one of or all of light with a wavelength from 620 nm to 670 nm (red), light with a wavelength from 450 nm to 480 nm (blue), light with a wavelength from 560 nm to 600 nm (yellow), and light with a wavelength from 500 nm to 530 nm (green), for example.

The term "cosmetic appliance" used in the present specification has meanings including a hair dryer for arranging a hairstyle, and a facial appliance (such as a facial roller and each of other various facial devices) for activating facial skin to create so-called "beautiful skin".

In the second through the fourth embodiments of the present invention, it is suitable that the multiple LEDs 1A are arranged in a closed shape formed by a curve (such as a circle and an ellipse), and light emitted by each of the LEDs 1A travels in a direction inclined toward the inner region of the shape.

Also, in the second through the fourth embodiments of the present invention, there is provided a spray device 215 that can be attached to and detached from the light irradiation device 220, and the spray device 215 may suitably include a liquid storage unit 15A, and a spray unit 15B that sprays a mist of liquid (such as liquid for so-called "beautiful hair") stored in the liquid storage unit 15A.

The cosmetic appliance 180 may suitably be a hair dryer.

The cosmetic appliance 280 may suitably be a facial appliance (such as a facial roller and each of other various facial devices).

With the abovementioned configuration, in the case of a hair dryer, in addition to warm air or cool air, light emitted by the multiple LEDs 1A provided in the light irradiation device 210, 220, or 230 is delivered to the scalp of a user, thereby improving cell activity, increasing fibroblasts, producing a growth factor, producing collagen, and increasing vascularization in the user's scalp, so as to exert a favorable influence on the skin tissue and hair and to place the scalp in favorable condition. Also, since each of the light irradiation devices 210, 220, and 230 is configured to be attachable to and detachable from the cosmetic appliances 180 and 280, the light irradiation devices 210, 220, and 230 that can be attached to a conventional hair dryer or the like can be easily produced. Therefore, the present invention is applicable without significantly modifying a conventional hair dryer or the like.

Further, the multiple LEDs 1A provided in the light irradiation devices 210, 220, and 230 are arranged in a circle (including an ellipse) and, if the light irradiation devices 210, 220, and 230 are configured so that light emitted by each of the LEDs 1A travels in a direction inclined radially inward of the circle, light emitted by the LEDs 1A will not reach an eye of a user of the cosmetic appliances 180 and 280 according to the present invention. This prevents the light emitted by the LEDs damaging the user's eyes.

In addition, when the spray device 215 is provided so as to be attachable to and detachable from the light irradiation device 220, and the spray device 215 is configured to include a spray unit that sprays a mist of liquid (such as liquid for so-called "beautiful hair") in the present invention, there can be obtained the effect of favorable hair condition due to the spraying of the liquid for beautiful hair, in addition to the effects of setting a hairstyle using warm air or cool air and the activation of the scalp by LED irradiation.

The present invention is applicable not only to a hair dryer but also to a facial appliance (such as a facial roller and each of other various facial devices).

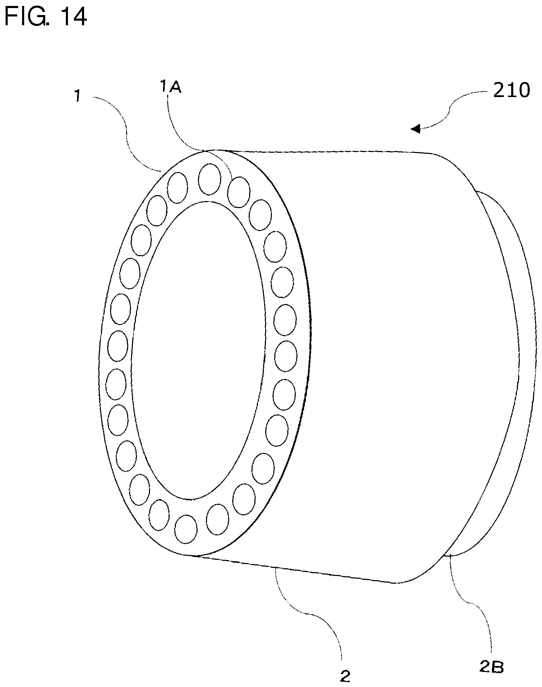

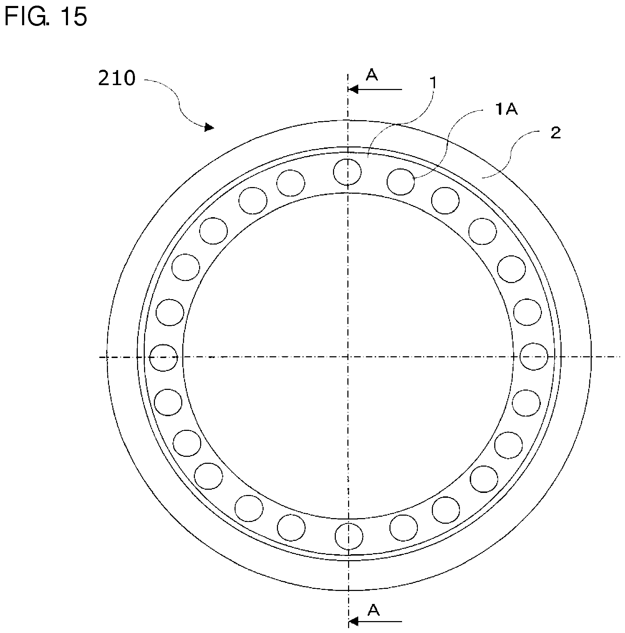

There will now be described the light irradiation device 210 according to the second embodiment of the present invention, with reference to Figs. 14-23. In the second embodiment, a hair dryer is used as an example of a cosmetic appliance. In Figs. 14 and 15, the light irradiation device 210 comprises a casing unit 2 of a substantially cylindrical shape formed of a synthetic resin material having electrical insulating properties, and an LED unit 1 including the multiple LEDs 1A arranged in a circular shape and provided on the front end (left side in Fig. 1) of the casing unit 2.

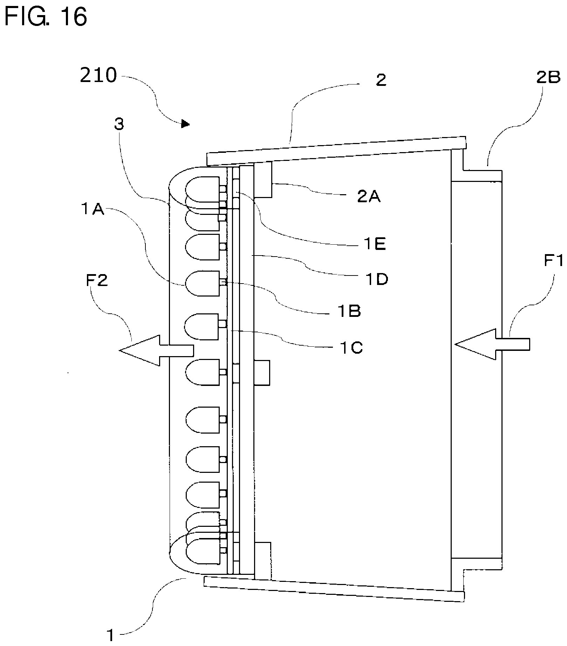

As shown in Fig. 16, to the front end (left end part in Fig. 14) of the casing unit 2, a cover 3 of a ring shape formed of a transparent synthetic resin material is attached, and the multiple LEDs 1A are covered with the cover 3. As will be described later with reference to Fig. 16, the LED unit 1 comprises the multiple LEDs 1A and a member for attaching the LEDs 1A to the front end of the casing unit 2.

The detailed configuration of the LED unit 1 will be described with reference to Figs. 15 and 16. In Fig. 16, the multiple LEDs 1A are provided on a mounting substrate 1C via the respective energization terminals 1B, and, between the LEDs 1A and the mounting substrate 1C, a thermal insulation board, not illustrated, is interposed. Each of the mounting substrate 1C, the thermal insulation board (not illustrated), and a supporting member 1D, which will be described later, is a ring-shaped plate, and the multiple LEDs 1A are substantially evenly spaced upon the mounting substrate 1C.

In Fig. 16, the mounting substrate 1C on which the multiple LEDs 1A are provided is fixed to the supporting member 1D via a spacer 1E. The supporting member 1D is fixed to an LED attachment part 2A provided near the front end (near the left end in Fig. 16) of the casing unit 2 formed in a substantially cylindrical shape. Specifically, the supporting member 1D is fixed to the LED attachment part 2A at multiple positions (four positions, for example) in a circumferential direction with well-known means, such as screws (not illustrated). The cover 3 of a ring shape formed of a transparent synthetic resin material is provided to cover the multiple LEDs 1A arranged in a ring and is attached to the supporting member 1D at multiple positions in an inner peripheral edge part and an outer peripheral edge part. Alternatively, the outer peripheral edge part of the cover 3 may be fixed to the front end part (left end part in Fig. 16) of the casing unit 2, and the inner peripheral edge part may be fixed to the supporting member 1D.

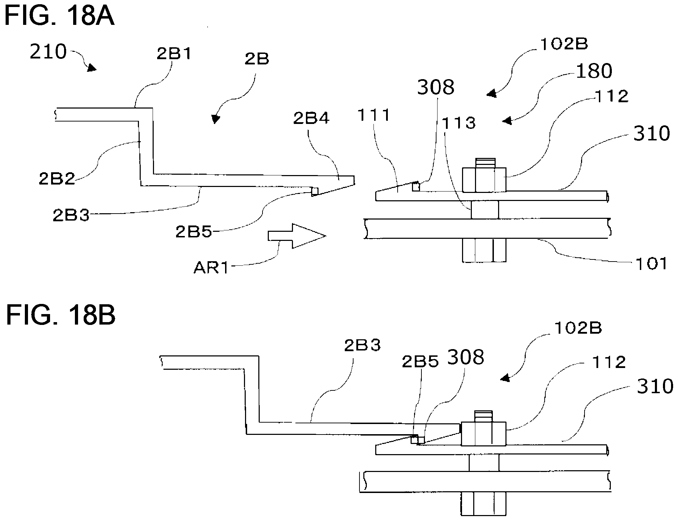

In Figs. 14 and 16, the rear end (right side in Figs. 14 and 16) of the casing unit 2 is a side attached to the cosmetic appliance 180, and an attachment part 2B for a hair dryer is formed near the rear end of the casing unit 2. The attachment part 2B for a hair dryer will be detailed later with reference to Figs. 18-20. When the light irradiation device 210 comprising the LED unit 1 and the casing unit 2 is attached near the air outlet of the cosmetic appliance 180 (not illustrated in Figs. 14-16), warm air or cool air from the cosmetic appliance 180 flows into the light irradiation device 210, as indicated by an arrow F1 in Fig. 16, and is provided from the LED unit 1 side of the light irradiation device 210 to the user's hair, as indicated by an arrow F2.

The multiple LEDs 1A may be selected from among LEDs that emit light with a wavelength from 620 nm to 670 nm (red), LEDs that emit light with a wavelength from 450 nm to 480 nm (blue), LEDs that emit light with a wavelength from 560 nm to 600 nm (yellow), and LEDs that emit light with a wavelength from 500 nm to 530 nm (green), in consideration of the effect of restoring skin provided by each of the LEDs. When selecting the LEDs 1A, LEDs in one of the abovementioned wavelength ranges may be selected, or LEDs in two or more of the abovementioned wavelength ranges may be selected, so as to be attached to the light irradiation device 210. Alternatively, LEDs in all of the abovementioned wavelength ranges may be selected to be attached to the light irradiation device 210.

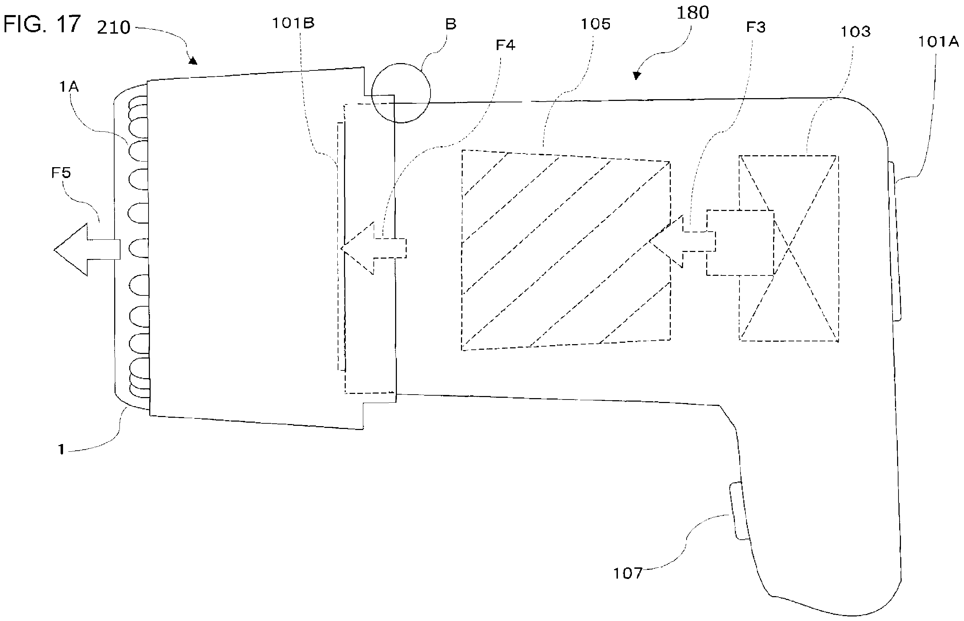

Fig. 17 shows the state where the light irradiation device 210 is attached to the cosmetic appliance 180.

In Fig. 17, the light irradiation device 210 as described with reference to Figs. 14-16 is attached near an air outlet 101B for warm air or cool air of the cosmetic appliance 180. The cosmetic appliance 180 shown in Fig. 17 comprises an attachment structure for the light irradiation device 210, and a mechanism for supplying electric power to the light irradiation device 210.

In Fig. 17, air is drawn in through an air inlet 101A by means of rotation of a blower fan 103 and then sent to a heater 105. In Fig. 17, air F3 sent to the heater 105 is indicated by an arrow. When a user of the cosmetic appliance 180 is to use warm air, the air F3 thus sent is heated by the heater 105, and, when the user is to use cool air, the air F3 is not heated. Thereafter, the heated or non-heated air (warm air or cool air: arrow F4) is provided through the air outlet 101B to the light irradiation device 210, passes through the inside (air passage) of the light irradiation device 210, and is then provided from the LED unit 1 side (left side in Fig. 17) toward the user's hair (arrow F5).

As shown in Fig. 17, with the cosmetic appliance 180 to which the light irradiation device 210 is attached, warm air or cool air is provided toward the user's hair (target of cosmetic treatment), and, at the same time, the scalp of the user is irradiated with light with a specific wavelength emitted by the LEDs 1A. Namely, the user can concurrently receive the effect of hair styling provided by the cosmetic appliance 180 and the effect of improving the scalp by LED irradiation provided by the light irradiation device 210, thereby efficiently acquiring the beauty effects. Also, supplying air by the cosmetic appliance 180 and light irradiation by the LEDs of the light irradiation device 210 can also be performed separately.

Although not illustrated, operating an operation switch part 107 of the cosmetic appliance 180 enables turning on and off of the light irradiation device 210, specification of an LED to be turned on (selection of a wavelength to be used), and selection and adjustment of strength of light emitted by the LED. In Fig. 17, an attachment section B is a section where the light irradiation device 210 is attached to the cosmetic appliance 180 and will be described below with reference to Figs. 18-20.

In Fig. 18A, the attachment part 2B is provided near the rear end part (near the right end part in Figs. 14, 16, and 17) of the cosmetic appliance 180. The attachment part 2B is integrally formed with the casing unit 2 and includes a body side part 2B1 of the casing unit 2, a vertical part 2B2 that extends from the body side part 2B1 radially inward and substantially vertically, and a horizontal part 2B3 that extends from the radially inner end part {the lower end in Fig. 18A} of the vertical part 2B2 in the axial direction (horizontally) toward the light irradiation device 210 side {the right side in Fig. 18A}. Near the rear end {right end in Fig. 18A} of the horizontal part 2B3, a protruding part 2B4 that protrudes radially inward is formed.

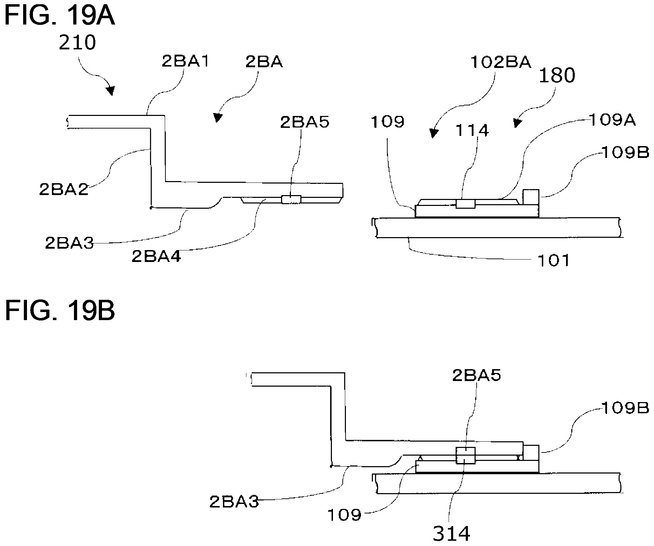

In the radially inner part {lower part in Fig. 18A} of the protruding part 2B4 is embedded an electrode 2B5, via which operating power is provided to the LED unit 1 of the light irradiation device 210. The attachment part 2B is provided at multiple positions (four positions, for example) that are substantially evenly spaced in a circumferential direction. Meanwhile, the electrode 2B5 may be provided in at least one position. Since the attachment part 2B is provided at multiple positions (four positions, for example) substantially evenly spaced in a circumferential direction, the horizontal parts 2B3 of the attachment parts 2B extend in an axial direction (the left or right direction in Figs. 18 and 19) in parts of the circumferential direction. Namely, the horizontal part 2B3 does not extend in an axial direction over the entire range of the circumferential direction.

In Fig. 18A, near the front end part {left end part in Fig. 18A} of the cosmetic appliance 180, an attachment part 102B of the cosmetic appliance 180 side is provided. Near the front end of a body case 101 and on the outer peripheral side thereof is provided a locking member 310, which extends substantially in parallel with an axial direction of the cosmetic appliance 180 by means of a fastening member 112. In the front end part {the light irradiation device 210 side} of the locking member 310, a protruding part 111 that protrudes radially outward is formed. Between the locking member 310 and the body case 101 is provided a spacer 113, so that, if the locking member 310 is elastically deformed in a radial direction when the light irradiation device 210 is attached, the displacement can be absorbed.

Near the front end {left end in Fig. 18A} of the locking member 310, an electrode 308 is embedded in the rear side {the right side in Fig. 18A} of the protruding part 111. Via a non-illustrated circuit, electric power from a power supply in the cosmetic appliance 180 is supplied to the electrode 308. The entire of the attachment part in the cosmetic appliance 180, including the locking member 310 and the like, is provided as the attachment part 102B. The attachment part 102B of the cosmetic appliance 180 is formed at multiple positions (four positions, for example) substantially evenly spaced in a circumferential direction so as to correspond to the attachment parts 2B of the light irradiation device 210.

In order to attach the light irradiation device 210 to the cosmetic appliance 180, the position of an attachment part 2B of the light irradiation device 210 is set to the position of an attachment part 102B of the cosmetic appliance 180, and the attachment part 2B of the light irradiation device 210 is inserted in the direction toward the attachment part 102B of the cosmetic appliance 180 {direction of an arrow AR1: the right side in Fig. 18A}. When the attachment part 2B of the light irradiation device 210 is inserted to the attachment part 102B of the cosmetic appliance 180, the protruding part 2B4 on the horizontal part 2B3 in the light irradiation device 210 resists elastic repulsion between the horizontal part 2B3 and the locking member 310 and passes over the protruding part 111 on the locking member 310 in the cosmetic appliance 180. Accordingly, the protruding part 2B4 is engaged with the protruding part 111, as shown in Fig. 18B. At the time, the upper edge part of the fastening member 112 in the cosmetic appliance 180 abuts onto the horizontal part 2B3 (protruding part 2B4), thereby preventing the horizontal part 2B3 (protruding part 2B4) excessively intruding into the cosmetic appliance 180 side (the right side in Figs. 18) (the upper edge part functions as a stopper).

As stated above, the part where the attachment part 2B and the attachment part 102B, which each extend in an axial direction (the left or right direction in Figs. 18), are engaged with each other is provided at multiple positions (four positions, for example) in a circumferential direction, so that the light irradiation device 210 can be attached to the cosmetic appliance 180. When the light irradiation device 210 is attached to the cosmetic appliance 180 in the state shown in Fig. 18B, the electrode 2B5 of the light irradiation device 210 abuts against the electrode 308 of the cosmetic appliance 180. In other words, since the electrode 2B5 of the light irradiation device 210 and the electrode 308 of the cosmetic appliance 180 are set so that the attachment part 2B and the attachment part 102B are engaged with and abut against each other, current flows between the electrode 2B5 and the electrode 308. Accordingly, electric power required for the LED unit 1 of the light irradiation device 210 to emit light is supplied from a power supply (an existing power supply) in the cosmetic appliance 180, through the electrode 308 of the cosmetic appliance 180 and the electrode 2B5 of the light irradiation device 210.

In order to detach the light irradiation device 210 from the cosmetic appliance 180, the user may hold, with fingers, the horizontal parts 2B3 of the attachment parts 2B of the light irradiation device 210 from the both sides in a circumferential direction and then move the protruding parts 2B4 on the horizontal parts 2B3 radially outward. The protruding parts 2B4 can be easily moved radially outward, and the attachment parts 2B and the attachment parts 102B are disengaged from each other, so that no current flows between the electrodes 2B5 and the electrodes 308, and the light irradiation device 210 is detached from the cosmetic appliance 180.

With reference to Figs. 19, a first modification of the structure for attaching the light irradiation device 210 to the cosmetic appliance 180 will be described. In Fig. 19A, an attachment part 2BA is integrally formed with the casing unit 2 near the rear end part {the right end part in Fig. 19A} of the light irradiation device 210 and includes a body side part 2BA1 of the casing unit 2, a vertical part 2BA2 that extends from the body side part 2BA1 radially inward and substantially vertically, and a horizontal part 2BA3 that substantially horizontally extends from the radially inner end {the lower end in Fig. 19A} of the vertical part 2BA2 in an axial direction of the light irradiation device 210 (the left or right direction in Figs. 19).

On the surface (inner peripheral surface) of the radially inner side {lower side in Fig. 19A} of the horizontal part 2BA3 and on the rear end side thereof {right side in Fig. 19A}, a female thread 2BA4 is formed.

On the female thread 2BA4, a notch (no symbol in the figures) is formed near the center in an axial direction, and an electrode 2BA5 is embedded in the notch. Via the electrode 2BA5, electric power required for the LED unit 1 of the light irradiation device 210 to emit light is supplied. The female thread 2BA4 is formed over the entire range of the circumferential direction on the attachment part 2BA, but the electrode 2BA5 need not be formed over the entire range of the circumferential direction. As will be described later, it may be sufficient as long as the electrode 2BA5 is formed to be certainly contactable with an electrode 314 of the cosmetic appliance 180.

In Fig. 19A, near the front end part {left end in Fig. 19A} of the cosmetic appliance 180, an attachment part 102BA of the cosmetic appliance 180 side is provided. Near the front end {left end in Fig. 19A} of the body case 101 of the cosmetic appliance 180 and on the outer peripheral side {upper side in Fig. 19A} thereof is provided a projection 109, and a male thread 109A is formed on the outer peripheral side of the projection 109. The outer peripheral surface {upper surface in Fig. 19A} of the projection 109 is substantially in parallel with an axial direction (the left or right direction in Figs. 19). In Fig. 19A, a protrusion 109B is a protrusion integrally formed with the projection 109 and functions as a stopper for the female thread 2BA4 when the light irradiation device 210 is attached.

On the male thread 109A, a notch (no symbol in the figures) is formed near the center in an axial direction, and an electrode 314 is embedded in the notch and connected to a power supply in the cosmetic appliance 180 via a non-illustrated circuit. The attachment part 102BA of the cosmetic appliance 180 is provided over the entire range of the circumference to correspond to the attachment part 2BA of the light irradiation device 210, whereas the electrode 314 is provided according to the position of the electrode 2BA5 of the light irradiation device 210. In other words, the electrode 314 is provided at a position that is certainly contactable with the electrode 2BA5 when the light irradiation device 210 is attached to the cosmetic appliance 180.

In Fig. 19A, in order to attach the light irradiation device 210 to the cosmetic appliance 180, the attachment part 2BA of the light irradiation device 210 is set to the attachment part 102BA of the cosmetic appliance 180, and the attachment part 2BA of the light irradiation device 210 is rotated so that the female thread 2BA4 is threadedly engaged with the male thread 109A. In this way, by threadedly engaging the female thread 2BA4 of the light irradiation device 210 and the male thread 109A of the cosmetic appliance 180, the light irradiation device 210 is attached to the cosmetic appliance 180 in the state shown in Fig. 19B.

When the light irradiation device 210 is attached to the cosmetic appliance 180 as shown in Fig. 19B, the electrode 2BA5 of the light irradiation device 210 is in contact with the electrode 314 of the cosmetic appliance 180, so that current flows between the electrode 2BA5 and the electrode 314. Accordingly, electric power required for the LED unit 1 of the light irradiation device 210 to emit light is supplied from a power supply in the cosmetic appliance 180, through the electrode 314 of the cosmetic appliance 180 and the electrode 2BA5 of the light irradiation device 210. In order to detach the light irradiation device 210 from the cosmetic appliance 180 in the state shown in Fig. 19B, the user may rotate the attachment part 2BA of the light irradiation device 210 so that the female thread 2BA4 and the male thread 109A are disengaged from each other.

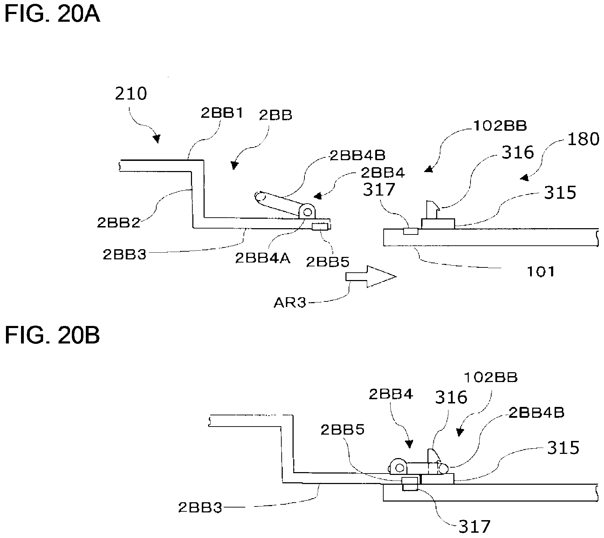

With reference to Figs. 20, a second modification of the structure for attaching the light irradiation device 210 to the cosmetic appliance 180 will be described. In Fig. 20A, an attachment part 2BB is integrally formed with the casing unit 2 near the rear end part {the right end side in Fig. 20A} of the light irradiation device 210 and includes a body side part 2BB1 of the casing unit 2, a vertical part 2BB2 that extends from the body side part 2BB1 radially inward and substantially vertically, and a horizontal part 2BB3 that horizontally extends from the radially inner end part {the lower end in Fig. 20A} of the vertical part 2BB2 in an axial direction of the light irradiation device 210 (the left or right direction in Figs. 20). Near an end part {the right end in Fig. 20A} of the horizontal part 2BB3, a locking member 2BB4, including a support shaft 2BB4A and a locking part 2BB4B of a rectangle ring shape are provided, and a locking member 2BB4 is rotatably supported by the support shaft 2BB4A at one end.

Near the rear end of the horizontal part 2BB3 and on the radially inner surface {lower surface in Fig. 20A} thereof is embedded an electrode 2BB5, via which electric power required for the LED unit 1 of the light irradiation device 210 to emit light is supplied. The attachment part 2BB is provided at multiple positions (four positions, for example) that are substantially evenly spaced in a circumferential direction. Meanwhile, the electrode 2BB5 may be provided in at least one position.

In Fig. 20A, the cosmetic appliance 180 is provided with an attachment part 102BB. Near the front end {left end in Fig. 20A} of the body case 101 and on the outer peripheral side {upper side in Fig. 20A} thereof is provided a projection 315, and, on the outer peripheral side {upper side in Fig. 20A} of the projection 315, a lock reception part 316 is provided for the locking member 2BB4 of the light irradiation device 210. To the side closer to the light irradiation device 210 (to the left side in Figs. 20) of the projection 315 and on the outer peripheral side {upper side in Fig. 20A} is embedded an electrode 317, to which electric power is supplied from a power supply in the cosmetic appliance 180 via a non-illustrated circuit.

The inner peripheral surface {lower surface in Fig. 20A} of the horizontal part 2BB3 in the light irradiation device 210 and the outer peripheral surface {upper surface in Fig. 20A} of the body case 101 in the cosmetic appliance 180 are set so as to be flush with each other when the light irradiation device 210 is attached to the cosmetic appliance 180. The size in a radial direction (thickness) of the projection 315 provided on the body case 101 is substantially identical with the size in a radial direction (thickness) of the horizontal part 2BB3 of the attachment part 2BB in the light irradiation device 210. When the light irradiation device 210 is attached to the cosmetic appliance 180, the projection 315 functions as a stopper for the horizontal part 2BB3 of the light irradiation device 210. The attachment part 102BB of the cosmetic appliance 180 is provided at multiple positions (four positions, for example), which correspond to the positions of the attachment parts 2BB of the light irradiation device 210, substantially evenly spaced in a circumferential direction.

When the light irradiation device 210 is to be attached to the cosmetic appliance 180, the position of an attachment part 2BB of the light irradiation device 210 is set to an attachment part 102BB of the cosmetic appliance 180. Subsequently, the attachment part 2BB is inserted toward the attachment part 102BB (in the direction of an arrow AR3) until the tip {right end in Fig. 20A} of the horizontal part 2BB3 abuts onto the projection 315. Thereafter, by rotationally moving, clockwise in Fig. 20A, the locking part 2BB4B of a rectangle ring shape of the locking member 2BB4 on the horizontal part 2BB3 so that the locking part 2BB4B is engaged with the lock reception part 316, the light irradiation device 210 can be attached to the cosmetic appliance 180 {the state shown in Fig. 20B}. In order to detach the light irradiation device 210 from the cosmetic appliance 180 in the state shown in Fig. 20B, the user may rotationally move the locking part 2BB4B counterclockwise in Fig. 20B so that the locking part 2BB4B is disengaged from the lock reception part 316.

In this way, by engaging the attachment parts 2BB of the light irradiation device 210 and the attachment parts of the cosmetic appliance 180 at multiple positions (four positions, for example), the light irradiation device 210 is certainly attached to the cosmetic appliance 180 in the state shown in Fig. 20B. When the light irradiation device 210 is attached to the cosmetic appliance 180 as shown in Fig. 20B, the electrode 2BB5 of the light irradiation device 210 is in contact with the electrode 317 of the cosmetic appliance 180, so that current flows between the electrode 2BB5 and the electrode 317. Accordingly, electric power required for the LED unit 1 of the light irradiation device 210 to emit light can be supplied from a power supply in the cosmetic appliance 180, through the electrode 317 of the cosmetic appliance 180 and the electrode 2BB5 of the light irradiation device 210.

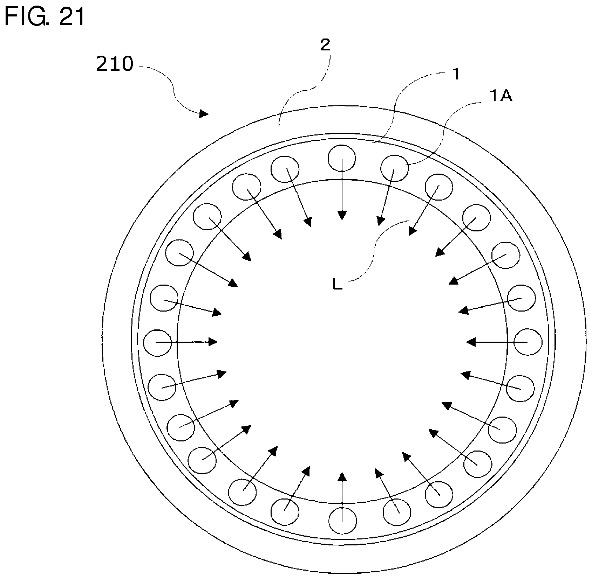

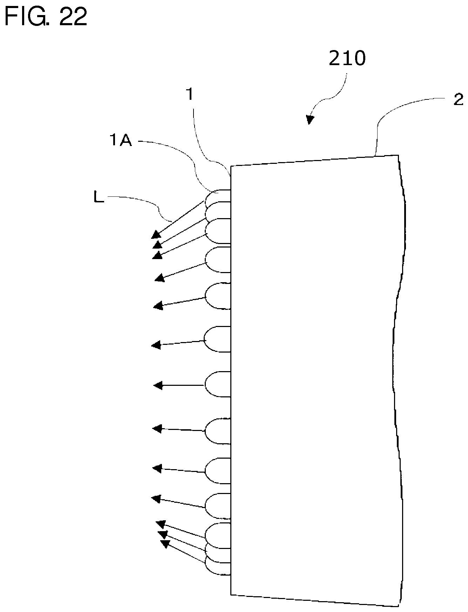

There will now be described the direction of light emitted by each of the LEDs of the light irradiation device 210 according to the second embodiment, with reference to Figs. 21, 22, and 23. As stated previously with reference to Figs. 14 and 15, the multiple LEDs 1A in the LED unit 1 are arranged in a circle to be substantially evenly spaced on the mounting substrate 1C, which is not illustrated. Each of the LEDs 1A is arranged so as to emit light in a direction inclined radially inward with respect to an axial direction of the light irradiation device 210 (the left or right direction in Figs. 16 and 22), and the light emitted by each of the LEDs 1A travels in a direction inclined radially inward with respect to an axial direction. In Figs. 21 and 22, the traveling direction of light emitted by each of the LEDs is indicated by an arrow L.

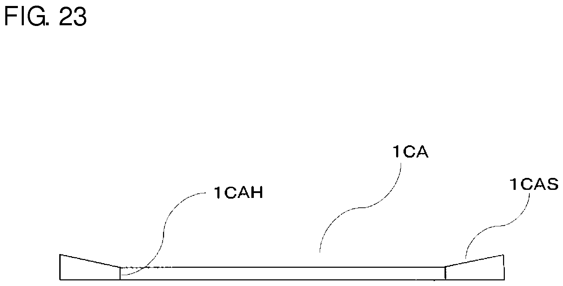

Although not clearly illustrated in Figs. 21 and 22, by adjusting the orientation of each of the LEDs 1A when arranging the LEDs 1A on the mounting substrate 1C (see Fig. 16), light emitted by each of the LEDs 1A can be set to travel in a direction inclined radially inward with respect to an axial direction. Alternatively, the mounting substrate on which the LEDs 1A are arranged may be formed in the shape of a mounting substrate 1CA shown in Fig. 23. The mounting substrate 1CA shown in Fig. 23 is not a ring-shaped flat plate, and an LED attachment surface 1CAS of a ring shape is formed to be thicker toward the outside in the radial direction, and hence, the LED attachment surface 1CAS for the LEDs 1A is inclined. In Fig. 23, a through hole 1CAH is a through hole. The configuration for setting the LEDs 1A to be inclined is not limited to that described above, and various configurations may be employed therefor.

According to the second embodiment shown in Figs. 14-23, in addition to warm air or cool air provided by the cosmetic appliance 180, light emitted by the multiple LEDs 1A provided in the light irradiation device 210 is delivered to the scalp of a user, so that the light emitted by the LEDs 1A, having a certain wavelength, improves cell activity, increases fibroblasts, produces a growth factor, produces collagen, and increases vascularization in the user's scalp, thereby exerting a favorable influence on the skin tissue and hair and placing the scalp in favorable condition.

Also, since the light irradiation device 210 is configured to be attachable to and detachable from the cosmetic appliance 180, the light irradiation device 210 can be easily applied to a conventional cosmetic appliance 180. In other words, the second embodiment is applicable without significantly modifying the conventional cosmetic appliance 180.

Also, according to the second embodiment as shown in the figures, since the multiple LEDs 1A provided in the light irradiation device 210 are arranged in a circle and the LEDs 1A are arranged so that light emitted by each of the LEDs 1A travels in a direction inclined toward a region radially inside the circle, light emitted by the LEDs 1A will not reach an eye of a user of the cosmetic appliance 180 according to the present invention. This prevents the light emitted by the LEDs 1A damaging the user's eyes.

In addition, according to the second embodiment as shown in the figures, when the light irradiation device 210 is attached to the cosmetic appliance 180, an electrode of the light irradiation device 210 abuts against an electrode of the cosmetic appliance 180, so that electric power can be supplied from the cosmetic appliance 180 to the light irradiation device 210; accordingly, electric power required for the LEDs 1A to emit light can be supplied from a power supply in the cosmetic appliance 180, without newly providing a power supply on the light irradiation device 210 side.

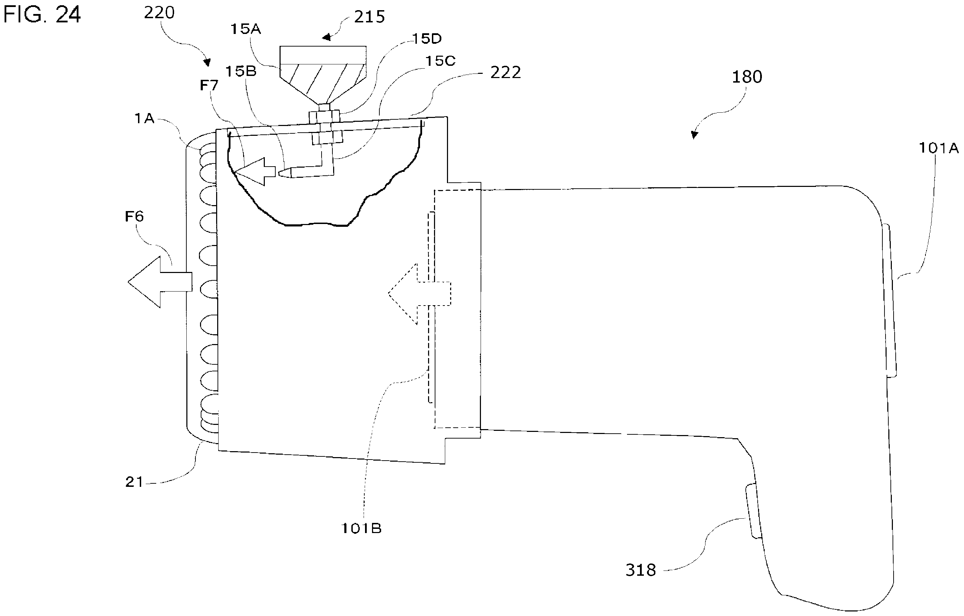

Next, a third embodiment of the present invention will be described with reference to Fig. 24.

In Fig. 24, to the air outlet 101B side (left side in Fig. 24) of the cosmetic appliance 180, the light irradiation device 220 according to the third embodiment is attached. The light irradiation device 220 is different from the light irradiation device 210 of the second embodiment in comprising the spray device 215, which is attachable and detachable, on the upper surface of a casing unit 222. Within the casing unit 222 of the light irradiation device 220, an upper space is made larger than a lower space or a side space so as to ensure space for the spray unit 15B of the spray device 215 and space for spraying. Accordingly, the shape of the light irradiation device 220 viewed from the left side in Fig. 24 is not an exact circle but a vertically-long ellipse. The other configurations of the light irradiation device 220 shown in Fig. 24 are the same as those of the light irradiation device 210 according to the second embodiment.

In Fig. 24, the spray device 215 has a function to spray a mist of liquid for so-called "beautiful hair", such as a hairdressing, within the casing unit 222. The spray device 215 comprises the liquid storage unit 15A provided on the upper side of the casing unit 222 in the light irradiation device 220, the spray unit 15B provided within the casing unit 222, and a communicating pipe 15C that communicates the liquid storage unit 15A and the spray unit 15B and that penetrates the upper surface of the casing unit 222. The spray device 215 is fixed to the upper surface of the casing unit 222 by a fastening member 15D. For the configuration of fixing the spray device 215 to the upper surface of the casing unit 222, a member other than the fastening member 15D may also be used. To the spray device 215, part of electric power supplied from a power supply in the cosmetic appliance 180 to the light irradiation device 220 is supplied via a non-illustrated circuit.

When the light irradiation device 220 is attached to the cosmetic appliance 180 and an operation switch part 318 of the cosmetic appliance 180 is operated to start the hair dryer, warm air or cool air passes through an airflow passage within the cosmetic appliance 180 and an air passage within the light irradiation device 220, and is then provided through the outlet of the light irradiation device 220 toward the user's hair (target of cosmetic treatment), as indicated by an arrow F6. In Fig. 24, when the operation switch part 318 is operated to start the spray device 215, a mist of liquid for beautiful hair is sprayed by the spray unit 15B of the spray device 215 into the air passage in the light irradiation device 220 (arrow F7).

The mist of liquid for beautiful hair sprayed by the spray device 215 is carried by the warm air or cool air provided by the cosmetic appliance 180 and efficiently delivered to the user's hair as the target of cosmetic treatment. Even when air is not provided by the cosmetic appliance 180, with the spraying power of the spray device 215, the liquid for beautiful hair stored in the spray device 215 can be delivered to the user's hair. Also, by turning on the LEDs 1A of the light irradiation device 220 in addition to spraying the liquid for beautiful hair from the spray device 215, the scalp of the user is irradiated with light with a specific wavelength emitted by the LEDs 1A, thereby also improving the condition of the user's scalp.

According to the third embodiment shown in Fig. 24, since the spray device 215 is provided to be attachable to and detachable from the light irradiation device 220 and the spray device 215 includes the spray unit for spraying a mist of liquid for beautiful hair, there can be obtained the effect of favorable hair condition due to the spraying of the liquid for beautiful hair, in addition to the effects of setting a hairstyle using warm air or cool air and the activation of the scalp by LED irradiation. Especially, by spraying the liquid for beautiful hair in the warm air or cool air provided by the cosmetic appliance 180, the mist of the liquid for beautiful hair is carried by the warm air or cool air from the cosmetic appliance 180 and efficiently delivered to the user's hair as the target of cosmetic treatment. The other configurations and effects of the third embodiment shown in Fig. 24 are the same as those of the second embodiment described with reference to Figs. 14-23.

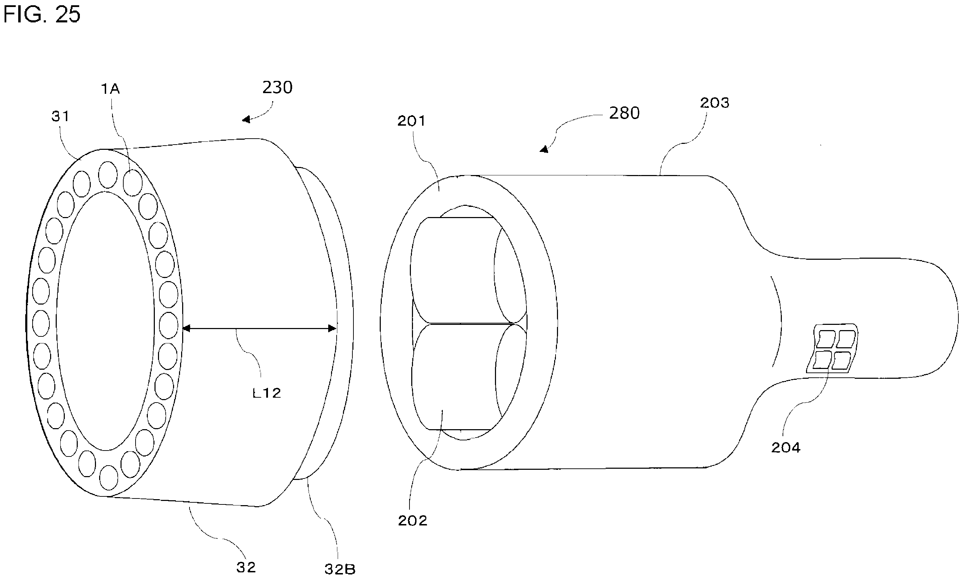

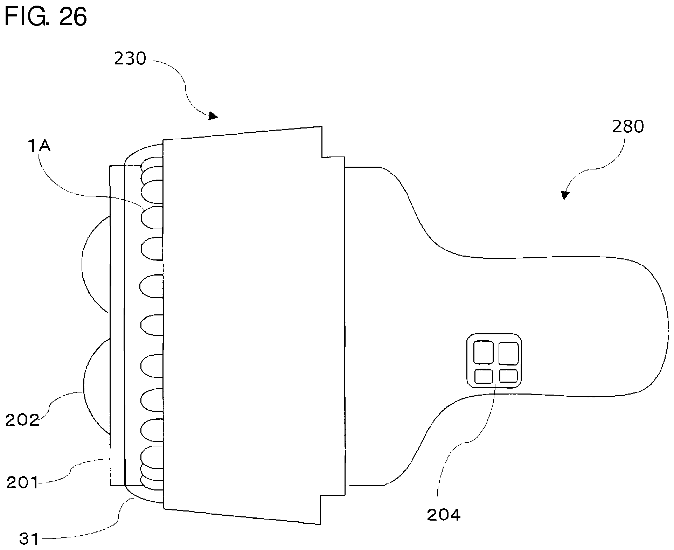

Next, a fourth embodiment of the present invention will be described with reference to Figs. 25 and 26.

Although the cosmetic appliance 180 in the second and third embodiments shown in Figs. 14-24 is a hair dryer, the cosmetic appliance 280 in the fourth embodiment shown in Figs. 25 and 26 is a facial roller (facial instrument). The cosmetic appliance 280 as a facial roller includes a pair of rollers 202 provided vertically on a head part 201, and the rollers 202 are rotated by means of a non-illustrated power supply so as to massage facial skin, providing the effects of stimulating blood flow and activating metabolism in the facial skin. In Figs. 25 and 26, an operation switch part 204 is an operation switch part.

In Fig. 25, a light irradiation device 230 is similar to the light irradiation device 210 of the second embodiment but different from the light irradiation device 210 in sizes, such as the inner and outer diameters and the length in a side surface direction, and the number of the LEDs 1A, for example, because the light irradiation device 230 is attached to the cosmetic appliance 280 instead of the cosmetic appliance 180. Since the rollers 202 of the cosmetic appliance 280 need to protrude from an LED unit 31 at the front end of the light irradiation device 230 when the light irradiation device 230 is attached to the cosmetic appliance 280 (see Fig. 26), a length L12 in an axial direction of the light irradiation device 230 (the left or right direction in Figs. 25 and 26) is set to be smaller than that in the embodiments shown in Figs. 14-24. An attachment mechanism 32B used to attach the light irradiation device 230 to the cosmetic appliance 280, and a mechanism for supplying electric power from the cosmetic appliance 280 are similar to those in the embodiments shown in Figs. 14-24.

In Fig. 26, which shows the state where the light irradiation device 230 is attached to the cosmetic appliance 280, the rollers 202 protrude from the light irradiation device 230 in the light irradiation direction (left direction in Fig. 26). In order to achieve the facial effect of the cosmetic appliance 280, the rollers 202 need to come into contact with the user's face; however, if the rollers 202 do not protrude from the light irradiation device 230 in the light irradiation direction (left direction in Fig. 26), the light irradiation device 230 will be in contact with the user's face, so that the rollers 202 are unable to come into contact with the user's face.

When the cosmetic appliance 280 is used, the operation switch part 204 is operated to start the light irradiation device 230, and light with a desired wavelength and strength is emitted from the LEDs, thereby activating the user's skin. Further, when the light irradiation device 230 is attached to the cosmetic appliance 280 to be used, as shown in Fig. 26, the cosmetic appliance 280 is stated to perform treatment of the face as the target of cosmetic treatment, and, subsequently, the scalp can be activated by allowing the light irradiation device 230 to emit light.

According to the fourth embodiment shown in Figs. 25 and 26, facial skin treatment can be performed for a user by means of the cosmetic appliance 280 and, in addition, the user's scalp can be irradiated with LED light emitted by the light irradiation device 230, thereby improving cell activity, increasing fibroblasts, producing a growth factor, producing collagen, and increasing vascularization in the user's scalp, so as to exert a favorable influence on the skin tissue and hair and to place the scalp in favorable condition. Therefore, the beauty effects can be synergistically improved. The other configurations and effects of the fourth embodiment shown in Figs. 25 and 26 are the same as those of the second embodiment described with reference to Figs. 14-23.

The present invention may also be understood with the following description.
(Claim 1) A light irradiation device comprising a plurality of light emitting diodes (LEDs) and configured to be attachable to and detachable from a cosmetic appliance.
(Claim 2) The light irradiation device of claim 1, wherein the plurality of LEDs are arranged in a closed shape formed by a curve, and light emitted by each of the LEDs travels in a direction inclined toward an inner region of the closed shape.
(Claim 3) The light irradiation device of claim 1 or 2, further comprising a spray device configured to be attachable and detachable and comprising liquid storage unit and a spray unit that sprays a mist of liquid stored in the liquid storage unit.

In the drawings used for the description, hatching is provided on the cross sections of part of the members in order to clarify the relationships between the members; however, such hatching is not provided to limit the materials of the members.

### [INDUSTRIAL APPLICABILITY]

The present invention has been made in view of such a problem, and a purpose thereof is to provide a technique of a light irradiation device for irradiating the scalp or the like of a user with light, in which the amount of light delivered to the scalp or the like can be increased while the influence of light that enters an eye is reduced.

### [EXPLANATION OF REFERENCE NUMERALS]

10 light emitting unit, 13 substrate,
14 electronic components, 15 hood member,
16 center hole part, 16b outlet, 18 cover member,
18a receding region, 18b projecting region,
19 projections, 19b recesses,
20 light emitting elements,
20a light emitting elements,
20b light emitting elements, 28 luminous flux,
30 housing, 32 current limiting unit,
32b limit switch, 34 sign output unit,
36 first contact part, 38 second contact part,
40 blower unit, 41 motor, 42 air flow,
43 impeller, 44 heater, 50 control unit,
52 switch unit, 70 irradiation target part,
82 first engagement part, 84 second engagement part,
86 vent hole, 100 light irradiation device,
108 grip portion, 110 casing, 114 air outlet,
116 air inlet, 118 wiring passage unit,
124 power supply unit, 126 electric cable,
128 electric cable

## Claims

1. A light irradiation device, comprising:
a blower unit that provides an air flow flowing in a first direction; and
a light emitting unit that outputs luminous flux to irradiate a scalp or hair as an irradiation target part, the light emitting unit being provided on the first direction side of the blower unit.

2. The light irradiation device of claim 1, wherein the blower unit is configured to provide the air flow toward the irradiation target part.

3. The light irradiation device of claim 1 or 2, wherein the light emitting unit includes a cover member that transmits the luminous flux and that is replaceable.

4. The light irradiation device of claim 3, wherein the cover member includes a plurality of projections projecting in the first direction.

5. The light irradiation device of claim 3 or 4, wherein the cover member includes a receding region that is receding toward the blower unit along the first direction, and a projecting region that is projecting forward of the receding region in the first direction.

6. The light irradiation device of any one of claims 1 through 5, wherein the light emitting unit comprises a hood member that surrounds luminous flux on the first direction side.

7. The light irradiation device of any one of claims 1 through 6, wherein the light emitting unit comprises a plurality of light emitting elements.

8. The light irradiation device of claim 7, wherein the plurality of light emitting elements include light emitting elements that are receding toward the blower unit along the first direction, and light emitting elements that are projecting forward of the receding light emitting elements in the first direction.

9. The light irradiation device of claim 7 or 8, wherein at least part of the plurality of light emitting elements are provided to be inclined with respect to the first direction.

10. The light irradiation device of any one of claims 7 through 9, wherein at least part of the plurality of light emitting elements are arranged in a region where the air flow passes through.

11. The light irradiation device of any one of claims 7 through 10, wherein, in the light emitting unit, at least part of electronic components, each electrically connected to one of the plurality of light emitting elements, are arranged in a range where the air flow passes through.

12. The light irradiation device of any one of claims 1 through 11, further comprising a current limiting unit configured to shut off or reduce a current supplied to the light emitting unit when the light emitting unit is apart from the irradiation target part by a predetermined distance or more.

13. The light irradiation device of any one of claims 1 through 12, further comprising a sign output unit configured to output a sign that can be perceived by a user at predetermined timing.

14. The light irradiation device of claim 13, wherein the sign output unit is configured to output a sign using at least one of sound, vibration, and light, at timing when a predetermined period of time elapses after the supply of a current to the light emitting unit is started.

15. The light irradiation device of any one of claims 1 through 14, wherein:
the light emitting unit is provided to be attachable to and detachable from the blower unit;
the blower unit includes a first contact part used to supply a current to the light emitting unit; and
the light emitting unit includes a second contact part that is electrically connected to the first contact part in the state where the light emitting unit is attached to the blower unit.
